# EUROPEAN PATENT SPECIFICATION

(11) **EP 3 293 520 B1**
(45) Date of publication and mention of the grant of the patent: **06.11.2019**
(21) Application number: 16001964.2
(22) Date of filing: 09.09.2016
(51) Int. Cl.: G01N 33/564

(54) **A METHOD FOR THE PRODUCTION OF AN POLYPEPTIDE**
VERFAHREN ZUR HERSTELLUNG EINES POLYPEPTIDS
PROCÉDÉ POUR LA PRODUCTION D'UN POLYPEPTIDE

(43) Date of publication of application: 14.03.2018
(73) Proprietor: Euroimmun Medizinische Labordiagnostika AG, 23560 Lübeck (DE)
(72) Inventor: Probst, Christian, 23909 Ratzeburg (DE); Dähnrich, Cornelia, 23627 Groß Grönau (DE); Komorowski, Lars, 23909 Ratzeburg (DE)

(56) References cited:
- WO-A1-2013/006059
- WO-A1-2015/023626
- MERRILEE NEEDHAM ET AL: "Sporadic inclusion body myositis: A review of recent clinical advances and current approaches to diagnosis and treatment", CLINICAL NEUROPHYSIOLOGY, vol. 127, no. 3, 19 December 2015 (2015-12-19), pages 1764-1773, XP055340100, IE ISSN: 1388-2457, DOI: 10.1016/j.clinph.2015.12.011
- H. BENJAMIN LARMAN ET AL: "Cytosolic 5'-nucleotidase 1A autoimmunity in sporadic inclusion body myositis", ANNALS OF NEUROLOGY., vol. 73, no. 3, 17 March 2013 (2013-03-17) , pages 408-418, XP055319183, BOSTON, US ISSN: 0364-5134, DOI: 10.1002/ana.23840
- SANDER H. J. DOOREN ET AL: "Myositis-specific autoantibodies: detection and clinical associations", AUTOIMMUNITY HIGHLIGHTS, vol. 2, no. 1, 23 March 2011 (2011-03-23), pages 5-20, XP055020327, ISSN: 2038-0305, DOI: 10.1007/s13317-011-0018-8

### SEQUENCE LISTING

<110> EUROIMMUN Medizinische Labordiagnostika AG
<120> A method for the production of a polypeptide
<130> 16PP080
<160> 16
<170> PatentIn version 3.5
<210> 1
   <211> 368
   <212> PRT
   <213> Homo sapiens
<400> 1
   Met Glu Pro Gly Gln Pro Arg Glu Pro Gln Glu Pro Arg Glu Pro Gly
   1 5 10 15
   Pro Gly Ala Glu Thr Ala Ala Ala Pro Val Trp Glu Glu Ala Lys Ile
   20 25 30
   Phe Tyr Asp Asn Leu Ala Pro Lys Lys Lys Pro Lys Ser Pro Lys Pro
   35 40 45
   Gln Asn Ala Val Thr Ile Ala Val Ser Ser Arg Ala Leu Phe Arg Met
   50 55 60
   Asp Glu Glu Gln Gln Ile Tyr Thr Glu Gln Gly Val Glu Glu Tyr Val
   65 70 75 80
   Arg Tyr Gln Leu Glu His Glu Asn Glu Pro Phe Ser Pro Gly Pro Ala
   85 90 95
   Phe Pro Phe Val Lys Ala Leu Glu Ala Val Asn Arg Arg Leu Arg Glu
   100 105 110
   Leu Tyr Pro Asp Ser Glu Asp Val Phe Asp Ile Val Leu Met Thr Asn
   115 120 125
   Asn His Ala Gln Val Gly Val Arg Leu Ile Asn Ser Ile Asn His Tyr
   130 135 140
   Asp Leu Phe Ile Glu Arg Phe Cys Met Thr Gly Gly Asn Ser Pro Ile
   145 150 155 160
   Cys Tyr Leu Lys Ala Tyr His Thr Asn Leu Tyr Leu Ser Ala Asp Ala
   165 170 175
   Glu Lys Val Arg Glu Ala Ile Asp Glu Gly Ile Ala Ala Ala Thr Ile
   180 185 190
   Phe Ser Pro Ser Arg Asp Val Val Val Ser Gln Ser Gln Leu Arg Val
   195 200 205
   Ala Phe Asp Gly Asp Ala Val Leu Phe Ser Asp Glu Ser Glu Arg Ile
   210 215 220
   Val Lys Ala His Gly Leu Asp Arg Phe Phe Glu His Glu Lys Ala His
   225 230 235 240
   Glu Asn Lys Pro Leu Ala Gln Gly Pro Leu Lys Gly Phe Leu Glu Ala
   245 250 255
   Leu Gly Arg Leu Gln Lys Lys Phe Tyr Ser Lys Gly Leu Arg Leu Glu
   260 265 270
   Cys Pro Ile Arg Thr Tyr Leu Val Thr Ala Arg Ser Ala Ala Ser Ser
   275 280 285
   Gly Ala Arg Ala Leu Lys Thr Leu Arg Ser Trp Gly Leu Glu Thr Asp
   290 295 300
   Glu Ala Leu Phe Leu Ala Gly Ala Pro Lys Gly Pro Leu Leu Glu Lys
   305 310 315 320
   Ile Arg Pro His Ile Phe Phe Asp Asp Gln Met Phe His Val Ala Gly
   325 330 335
   Ala Gln Glu Met Gly Thr Val Ala Ala His Val Pro Tyr Gly Val Ala
   340 345 350
   Gln Thr Pro Arg Arg Thr Ala Pro Ala Lys Gln Ala Pro Ser Ala Gln
   355 360 365
<210> 2
   <211> 23
   <212> PRT
   <213> artificial
<220>
   <223> major epitope 1 of cN-1A
<400> 2
   Pro Val Trp Glu Glu Ala Lys Ile Phe Tyr Asp Asn Leu Ala Pro Lys
   1 5 10 15
   Lys Lys Pro Lys Ser Pro Lys
   20
<210> 3
   <211> 23
   <212> PRT
   <213> artificial
<220>
   <223> major epitope 2 of cN-1A
<400> 3
   Ser Glu Arg Ile Val Lys Ala His Gly Leu Asp Arg Phe Phe Glu His
   1               5                   10                  15
   Glu Lys Ala His Glu Asn Lys
               20
<210> 4
   <211> 23
   <212> PRT
   <213> artificial
<220>
   <223> major epitope 3 of cN-1A
<400> 4
   Ala His Val Pro Tyr Gly Val Ala Gln Thr Pro Arg Arg Thr Ala Pro
   1               5                   10                  15
   Ala Lys Gln Ala Pro Ser Ala
               20
<210> 5
   <211> 376
   <212> PRT
   <213> artificial
<220>
   <223> immunogenic wild type sequence of cN-1A having a lower degree of enzymatic activity
<400> 5
   Met Glu Pro Gly Gln Pro Arg Glu Pro Gln Glu Pro Arg Glu Pro Gly
   1               5                   10                  15
   Pro Gly Ala Glu Thr Ala Ala Ala Pro Val Trp Glu Glu Ala Lys Ile
               20                  25                  30
   Phe Tyr Asp Asn Leu Ala Pro Lys Lys Lys Pro Lys Ser Pro Lys Pro
           35                  40                  45
   Gln Asn Ala Val Thr Ile Ala Val Ser Ser Arg Ala Leu Phe Arg Met
       50                  55                  60
   Asp Glu Glu Gln Gln Ile Tyr Thr Glu Gln Gly Val Glu Glu Tyr Val
   65                  70                  75                  80
   Arg Tyr Gln Leu Glu His Glu Asn Glu Pro Phe Ser Pro Gly Pro Ala
                   85                  90                  95
   Phe Pro Phe Val Lys Ala Leu Glu Ala Val Asn Arg Arg Leu Arg Glu
               100                 105                 110
   Leu Tyr Pro Asp Ser Glu Asp Val Phe Asp Ile Val Leu Met Thr Asn
           115                 120                 125
   Asn His Ala Gln Val Gly Val Arg Leu Ile Asn Ser Ile Asn His Tyr
       130                 135                 140
   Asp Leu Phe Ile Glu Arg Phe Cys Met Thr Gly Gly Asn Ser Pro Ile
   145                 150                 155                 160
   Cys Tyr Leu Lys Ala Tyr His Thr Asn Leu Tyr Leu Ser Ala Asp Ala
                   165                 170                 175
   Glu Lys Val Arg Glu Ala Ile Asp Glu Gly Ile Ala Ala Ala Thr Ile
               180                 185                 190
   Phe Ser Pro Ser Arg Asp Val Val Val Ser Gln Ser Gln Leu Arg Val
           195                 200                 205
   Ala Phe Ala Gly Asp Ala Val Leu Phe Ser Asp Glu Ser Glu Arg Ile
       210                 215                 220
   Val Lys Ala His Gly Leu Asp Arg Phe Phe Glu His Glu Lys Ala His
   225                 230                 235                 240
   Glu Asn Lys Pro Leu Ala Gln Gly Pro Leu Lys Gly Phe Leu Glu Ala
                   245                 250                 255
   Leu Gly Arg Leu Gln Lys Lys Phe Tyr Ser Lys Gly Leu Arg Leu Glu
               260                 265                 270
   Cys Pro Ile Arg Thr Tyr Leu Val Thr Ala Arg Ser Ala Ala Ser Ser
           275                 280                 285
   Gly Ala Arg Ala Leu Lys Thr Leu Arg Ser Trp Gly Leu Glu Thr Asp
       290                 295                 300
   Glu Ala Leu Phe Leu Ala Gly Ala Pro Lys Gly Pro Leu Leu Glu Lys
   305                 310                 315                 320
   Ile Arg Pro His Ile Phe Phe Asp Asp Gln Met Phe His Val Ala Gly
                   325                 330                 335
   Ala Gln Glu Met Gly Thr Val Ala Ala His Val Pro Tyr Gly Val Ala
               340                 345                 350
   Gln Thr Pro Arg Arg Thr Ala Pro Ala Lys Gln Ala Pro Ser Ala Gln
           355                 360                 365
   Leu Glu His His His His His His
       370                 375
<210> 6
   <211> 1104
   <212> DNA
   <213> artificial
<220>
   <223> codon-optimized nucleic acid encoding sequence of cN-1A
<400> 6
   atggaaccag gtcaacctag agaacctcag gaacctaggg aaccaggtcc aggagctgaa      60
   acagctgctg ctccagtgtg ggaggaagcg aaaatcttct acgacaatct ggcacccaag     120
   aagaaaccca agagtccgaa accccagaat gccgtgacaa ttgccgtgtc aagcagggct     180
   ctgttccgaa tggacgaaga acagcagatc tacaccgagc aaggcgtgga agaatacgtt     240
   cgctatcagc tcgaacacga aaatgaaccg ttcagccctg gacccgcatt tccctttgtt     300
   aaagccctgg aagcggtcaa caggagactg agggagcttt accctgattc tgaggacgtc     360
   tttgacatcg tgctgatgac aaacaaccat gctcaggtgg gagttcgcct gatcaactcc     420
   atcaaccact atgacctgtt tatagagcgc ttttgcatga ccggtggaaa ctccccaatt     480
   tgctacctca aagcctatca caccaatctg tacctttccg ctgatgccga gaaggtccga     540
   gaggcaatcg atgagggtat tgcagcagct acgatcttca gtccatccag agatgtcgtg     600
   gtgtctcaaa gccagctgag agtggcgttt gatggagatg ccgtactctt cagcgacgag     660
   tcagagcgta ttgtgaaggc acatgggctc gaccggttct ttgagcatga gaaggcccac     720
   gagaataagc ctcttgccca aggacctctg aaagggtttc tggaggcact gggcaggctg     780
   cagaagaagt tctatagcaa aggtttgcgc ttggagtgtc caatacggac ctatctcgtc     840
   acagctagat ctgctgcaag cagtggagcc cgagctctga aaacgttgcg ttcatggggc     900
   cttgagactg acgaggctct ctttctggct ggagctccca aaggtcctct gttggagaag     960
   atacggcctc acattttctt cgatgaccag atgttccacg ttgcaggtgc tcaggagatg    1020
   ggcaccgtag ccgctcatgt gccatacgga gtagcgcaaa ctcccaggag aactgcacca    1080
   gccaagcagg caccctctgc acag                                           1104
<210> 7
   <211> 6337
   <212> DNA
   <213> artificial
<220>
   <223> pET24d-Mup44
<400> 7
   ggggaattgt gagcggataa caattcccct ctagaaataa ttttgtttaa ctttaagaag      60
   gagatatacc atggaacctg ggcagccccg ggagccccag gagccccgcg agcccgggcc     120
   aggagcggag accgctgcgg ccccggtctg ggaggaagcc aagattttct acgacaacct     180
   cgcgcccaag aagaaaccca aatcgcccaa gcctcagaat gcagtcacca tcgctgtgtc     240
   ctcccgagcc ttgtttcgca tggacgagga gcagcagatc tacacggagc agggcgtgga     300
   ggagtacgtg cgctaccagc tggaacatga gaacgaaccc ttcagtcccg ggccagcctt     360
   cccttttgtg aaggctctgg aggccgtgaa caggcggctg cgggagctgt accctgatag    420
   tgaggacgtc ttcgacatcg tcctcatgac taacaaccat gctcaagtgg gtgtccgcct    480
   catcaacagt atcaaccact atgacctgtt catcgagagg ttctgcatga caggtgggaa    540
   cagcccgatc tgctacctca aggcctatca caccaacctc tacttgtcag ccgatgcgga    600
   aaaagtgcga gaagccattg atgaggggat cgcagctgcc accatcttca gccccagcag    660
   ggatgtggtt gtgtcccaga gtcagctgcg cgtggccttc gatggggacg ccgtgctctt    720
   ctcggacgag tcggagcgca tcgtcaaggc ccacgggctg gaccgattct tcgagcatga    780
   gaaggcccac gagaacaaac ctctggctca gggcccctta aagggctttc tggaggcact    840
   gggtaggttg cagaagaagt tctactccaa aggcctgcgg ctggagtgcc caattcgtac    900
   ctacttggtg acagcacgca gtgcagccag ttccggggcc cgggctctca agaccctgcg    960
   cagctggggc ctggagacag atgaagcctt gttccttgct ggagcgccca agggccctct   1020
   ccttgagaag atccgcccac acatcttctt tgatgaccag atgttccatg tggctggggc   1080
   tcaggagatg ggcactgtgg ccgcccatgt gccttatggt gtggcacaga caccccggcg   1140
   gactgcacct gcaaagcagg ccccatctgc acagctcgag caccaccacc accaccactg   1200
   agatccggct gctaacaaag cccgaaagga agctgagttg gctgctgcca ccgctgagca   1260
   ataactagca taaccccttg gggcctctaa acgggtcttg aggggttttt tgctgaaagg   1320
   aggaactata tccggattgg cgaatgggac gcgccctgta gcggcgcatt aagcgcggcg   1380
   ggtgtggtgg ttacgcgcag cgtgaccgct acacttgcca gcgccctagc gcccgctcct   1440
   ttcgctttct tcccttcctt tctcgccacg ttcgccggct ttccccgtca agctctaaat   1500
   cgggggctcc ctttagggtt ccgatttagt gctttacggc acctcgaccc caaaaaactt   1560
   gattagggtg atggttcacg tagtgggcca tcgccctgat agacggtttt tcgccctttg   1620
   acgttggagt ccacgttctt taatagtgga ctcttgttcc aaactggaac aacactcaac   1680
   cctatctcgg tctattcttt tgatttataa gggattttgc cgatttcggc ctattggtta   1740
   aaaaatgagc tgatttaaca aaaatttaac gcgaatttta acaaaatatt aacgtttaca   1800
   atttcaggtg gcacttttcg gggaaatgtg cgcggaaccc ctatttgttt atttttctaa   1860
   atacattcaa atatgtatcc gctcatgaat taattcttag aaaaactcat cgagcatcaa   1920
   atgaaactgc aatttattca tatcaggatt atcaatacca tatttttgaa aaagccgttt   1980
   ctgtaatgaa ggagaaaact caccgaggca gttccatagg atggcaagat cctggtatcg   2040
   gtctgcgatt ccgactcgtc caacatcaat acaacctatt aatttcccct cgtcaaaaat   2100
   aaggttatca agtgagaaat caccatgagt gacgactgaa tccggtgaga atggcaaaag   2160
   tttatgcatt tctttccaga cttgttcaac aggccagcca ttacgctcgt catcaaaatc   2220
   actcgcatca accaaaccgt tattcattcg tgattgcgcc tgagcgagac gaaatacgcg   2280
   atcgctgtta aaaggacaat tacaaacagg aatcgaatgc aaccggcgca ggaacactgc   2340
   cagcgcatca acaatatttt cacctgaatc aggatattct tctaatacct ggaatgctgt   2400
   tttcccgggg atcgcagtgg tgagtaacca tgcatcatca ggagtacgga taaaatgctt 2460
   gatggtcgga agaggcataa attccgtcag ccagtttagt ctgaccatct catctgtaac 2520
   atcattggca acgctacctt tgccatgttt cagaaacaac tctggcgcat cgggcttccc 2580
   atacaatcga tagattgtcg cacctgattg cccgacatta tcgcgagccc atttataccc 2640
   atataaatca gcatccatgt tggaatttaa tcgcggccta gagcaagacg tttcccgttg 2700
   aatatggctc ataacacccc ttgtattact gtttatgtaa gcagacagtt ttattgttca 2760
   tgaccaaaat cccttaacgt gagttttcgt tccactgagc gtcagacccc gtagaaaaga 2820
   tcaaaggatc ttcttgagat cctttttttc tgcgcgtaat ctgctgcttg caaacaaaaa 2880
   aaccaccgct accagcggtg gtttgtttgc cggatcaaga gctaccaact ctttttccga 2940
   aggtaactgg cttcagcaga gcgcagatac caaatactgt ccttctagtg tagccgtagt 3000
   taggccacca cttcaagaac tctgtagcac cgcctacata cctcgctctg ctaatcctgt 3060
   taccagtggc tgctgccagt ggcgataagt cgtgtcttac cgggttggac tcaagacgat 3120
   agttaccgga taaggcgcag cggtcgggct gaacgggggg ttcgtgcaca cagcccagct 3180
   tggagcgaac gacctacacc gaactgagat acctacagcg tgagctatga gaaagcgcca 3240
   cgcttcccga agggagaaag gcggacaggt atccggtaag cggcagggtc ggaacaggag 3300
   agcgcacgag ggagcttcca gggggaaacg cctggtatct ttatagtcct gtcgggtttc 3360
   gccacctctg acttgagcgt cgatttttgt gatgctcgtc aggggggcgg agcctatgga 3420
   aaaacgccag caacgcggcc tttttacggt tcctggcctt ttgctggcct tttgctcaca 3480
   tgttctttcc tgcgttatcc cctgattctg tggataaccg tattaccgcc tttgagtgag 3540
   ctgataccgc tcgccgcagc cgaacgaccg agcgcagcga gtcagtgagc gaggaagcgg 3600
   aagagcgcct gatgcggtat tttctcctta cgcatctgtg cggtatttca caccgcatat 3660
   atggtgcact ctcagtacaa tctgctctga tgccgcatag ttaagccagt atacactccg 3720
   ctatcgctac gtgactgggt catggctgcg ccccgacacc cgccaacacc cgctgacgcg 3780
   ccctgacggg cttgtctgct cccggcatcc gcttacagac aagctgtgac cgtctccggg 3840
   agctgcatgt gtcagaggtt ttcaccgtca tcaccgaaac gcgcgaggca gctgcggtaa 3900
   agctcatcag cgtggtcgtg aagcgattca cagatgtctg cctgttcatc cgcgtccagc 3960
   tcgttgagtt tctccagaag cgttaatgtc tggcttctga taaagcgggc catgttaagg 4020
   gcggtttttt cctgtttggt cactgatgcc tccgtgtaag ggggatttct gttcatgggg 4080
   gtaatgatac cgatgaaacg agagaggatg ctcacgatac gggttactga tgatgaacat 4140
   gcccggttac tggaacgttg tgagggtaaa caactggcgg tatggatgcg gcgggaccag 4200
   agaaaaatca ctcagggtca atgccagcgc ttcgttaata cagatgtagg tgttccacag 4260
   ggtagccagc agcatcctgc gatgcagatc cggaacataa tggtgcaggg cgctgacttc 4320
   cgcgtttcca gactttacga aacacggaaa ccgaagacca ttcatgttgt tgctcaggtc 4380
   gcagacgttt tgcagcagca gtcgcttcac gttcgctcgc gtatcggtga ttcattctgc 4440
   taaccagtaa ggcaaccccg ccagcctagc cgggtcctca acgacaggag cacgatcatg 4500
   cgcacccgtg gggccgccat gccggcgata atggcctgct tctcgccgaa acgtttggtg 4560
   gcgggaccag tgacgaaggc ttgagcgagg gcgtgcaaga ttccgaatac cgcaagcgac 4620
   aggccgatca tcgtcgcgct ccagcgaaag cggtcctcgc cgaaaatgac ccagagcgct 4680
   gccggcacct gtcctacgag ttgcatgata aagaagacag tcataagtgc ggcgacgata 4740
   gtcatgcccc gcgcccaccg gaaggagctg actgggttga aggctctcaa gggcatcggt 4800
   cgagatcccg gtgcctaatg agtgagctaa cttacattaa ttgcgttgcg ctcactgccc 4860
   gctttccagt cgggaaacct gtcgtgccag ctgcattaat gaatcggcca acgcgcgggg 4920
   agaggcggtt tgcgtattgg gcgccagggt ggtttttctt ttcaccagtg agacgggcaa 4980
   cagctgattg cccttcaccg cctggccctg agagagttgc agcaagcggt ccacgctggt 5040
   ttgccccagc aggcgaaaat cctgtttgat ggtggttaac ggcgggatat aacatgagct 5100
   gtcttcggta tcgtcgtatc ccactaccga gatatccgca ccaacgcgca gcccggactc 5160
   ggtaatggcg cgcattgcgc ccagcgccat ctgatcgttg gcaaccagca tcgcagtggg 5220
   aacgatgccc tcattcagca tttgcatggt ttgttgaaaa ccggacatgg cactccagtc 5280
   gccttcccgt tccgctatcg gctgaatttg attgcgagtg agatatttat gccagccagc 5340
   cagacgcaga cgcgccgaga cagaacttaa tgggcccgct aacagcgcga tttgctggtg 5400
   acccaatgcg accagatgct ccacgcccag tcgcgtaccg tcttcatggg agaaaataat 5460
   actgttgatg ggtgtctggt cagagacatc aagaaataac gccggaacat tagtgcaggc 5520
   agcttccaca gcaatggcat cctggtcatc cagcggatag ttaatgatca gcccactgac 5580
   gcgttgcgcg agaagattgt gcaccgccgc tttacaggct tcgacgccgc ttcgttctac 5640
   catcgacacc accacgctgg cacccagttg atcggcgcga gatttaatcg ccgcgacaat 5700
   ttgcgacggc gcgtgcaggg ccagactgga ggtggcaacg ccaatcagca acgactgttt 5760
   gcccgccagt tgttgtgcca cgcggttggg aatgtaattc agctccgcca tcgccgcttc 5820
   cactttttcc cgcgttttcg cagaaacgtg gctggcctgg ttcaccacgc gggaaacggt 5880
   ctgataagag acaccggcat actctgcgac atcgtataac gttactggtt tcacattcac 5940
   caccctgaat tgactctctt ccgggcgcta tcatgccata ccgcgaaagg ttttgcgcca 6000
   ttcgatggtg tccgggatct cgacgctctc ccttatgcga ctcctgcatt aggaagcagc 6060
   ccagtagtag gttgaggccg ttgagcaccg ccgccgcaag gaatggtgca tgcaaggaga 6120
   tggcgcccaa cagtcccccg gccacggggc ctgccaccat acccacgccg aaacaagcgc 6180
   tcatgagccc gaagtggcga gcccgatctt ccccatcggt gatgtcggcg atataggcgc 6240
   cagcaaccgc acctgtggcg ccggtgatgc cggccacgat gcgtccggcg tagaggatcg 6300
   agatctcgat cccgcgaaat taatacgact cactata 6337
<210> 8
   <211> 6337
   <212> DNA
   <213> artificial
<220>
   <223> pET24d-Mup44 [D211A]
<400> 8
   ggggaattgt gagcggataa caattcccct ctagaaataa ttttgtttaa ctttaagaag      60
   gagatatacc atggaacctg ggcagccccg ggagccccag gagccccgcg agcccgggcc     120
   aggagcggag accgctgcgg ccccggtctg ggaggaagcc aagattttct acgacaacct     180
   cgcgcccaag aagaaaccca aatcgcccaa gcctcagaat gcagtcacca tcgctgtgtc     240
   ctcccgagcc ttgtttcgca tggacgagga gcagcagatc tacacggagc agggcgtgga     300
   ggagtacgtg cgctaccagc tggaacatga gaacgaaccc ttcagtcccg ggccagcctt     360
   cccttttgtg aaggctctgg aggccgtgaa caggcggctg cgggagctgt accctgatag     420
   tgaggacgtc ttcgacatcg tcctcatgac taacaaccat gctcaagtgg gtgtccgcct     480
   catcaacagt atcaaccact atgacctgtt catcgagagg ttctgcatga caggtgggaa     540
   cagcccgatc tgctacctca aggcctatca caccaacctc tacttgtcag ccgatgcgga     600
   aaaagtgcga gaagccattg atgaggggat cgcagctgcc accatcttca gccccagcag     660
   ggatgtggtt gtgtcccaga gtcagctgcg cgtggccttc gcaggggacg ccgtgctctt     720
   ctcggacgag tcggagcgca tcgtcaaggc ccacgggctg gaccgattct tcgagcatga     780
   gaaggcccac gagaacaaac ctctggctca gggcccctta aagggctttc tggaggcact     840
   gggtaggttg cagaagaagt tctactccaa aggcctgcgg ctggagtgcc caattcgtac     900
   ctacttggtg acagcacgca gtgcagccag ttccggggcc cgggctctca agaccctgcg     960
   cagctggggc ctggagacag atgaagcctt gttccttgct ggagcgccca agggccctct    1020
   ccttgagaag atccgcccac acatcttctt tgatgaccag atgttccatg tggctggggc    1080
   tcaggagatg ggcactgtgg ccgcccatgt gccttatggt gtggcacaga caccccggcg    1140
   gactgcacct gcaaagcagg ccccatctgc acagctcgag caccaccacc accaccactg    1200
   agatccggct gctaacaaag cccgaaagga agctgagttg gctgctgcca ccgctgagca    1260
   ataactagca taaccccttg gggcctctaa acgggtcttg aggggttttt tgctgaaagg    1320
   aggaactata tccggattgg cgaatgggac gcgccctgta gcggcgcatt aagcgcggcg    1380
   ggtgtggtgg ttacgcgcag cgtgaccgct acacttgcca gcgccctagc gcccgctcct    1440
   ttcgctttct tcccttcctt tctcgccacg ttcgccggct ttccccgtca agctctaaat    1500
   cgggggctcc ctttagggtt ccgatttagt gctttacggc acctcgaccc caaaaaactt    1560
   gattagggtg atggttcacg tagtgggcca tcgccctgat agacggtttt tcgccctttg    1620
   acgttggagt ccacgttctt taatagtgga ctcttgttcc aaactggaac aacactcaac    1680
   cctatctcgg tctattcttt tgatttataa gggattttgc cgatttcggc ctattggtta    1740
   aaaaatgagc tgatttaaca aaaatttaac gcgaatttta acaaaatatt aacgtttaca    1800
   atttcaggtg gcacttttcg gggaaatgtg cgcggaaccc ctatttgttt atttttctaa    1860
   atacattcaa atatgtatcc gctcatgaat taattcttag aaaaactcat cgagcatcaa 1920
   atgaaactgc aatttattca tatcaggatt atcaatacca tatttttgaa aaagccgttt 1980
   ctgtaatgaa ggagaaaact caccgaggca gttccatagg atggcaagat cctggtatcg 2040
   gtctgcgatt ccgactcgtc caacatcaat acaacctatt aatttcccct cgtcaaaaat 2100
   aaggttatca agtgagaaat caccatgagt gacgactgaa tccggtgaga atggcaaaag 2160
   tttatgcatt tctttccaga cttgttcaac aggccagcca ttacgctcgt catcaaaatc 2220
   actcgcatca accaaaccgt tattcattcg tgattgcgcc tgagcgagac gaaatacgcg 2280
   atcgctgtta aaaggacaat tacaaacagg aatcgaatgc aaccggcgca ggaacactgc 2340
   cagcgcatca acaatatttt cacctgaatc aggatattct tctaatacct ggaatgctgt 2400
   tttcccgggg atcgcagtgg tgagtaacca tgcatcatca ggagtacgga taaaatgctt 2460
   gatggtcgga agaggcataa attccgtcag ccagtttagt ctgaccatct catctgtaac 2520
   atcattggca acgctacctt tgccatgttt cagaaacaac tctggcgcat cgggcttccc 2580
   atacaatcga tagattgtcg cacctgattg cccgacatta tcgcgagccc atttataccc 2640
   atataaatca gcatccatgt tggaatttaa tcgcggccta gagcaagacg tttcccgttg 2700
   aatatggctc ataacacccc ttgtattact gtttatgtaa gcagacagtt ttattgttca 2760
   tgaccaaaat cccttaacgt gagttttcgt tccactgagc gtcagacccc gtagaaaaga 2820
   tcaaaggatc ttcttgagat cctttttttc tgcgcgtaat ctgctgcttg caaacaaaaa 2880
   aaccaccgct accagcggtg gtttgtttgc cggatcaaga gctaccaact ctttttccga 2940
   aggtaactgg cttcagcaga gcgcagatac caaatactgt ccttctagtg tagccgtagt 3000
   taggccacca cttcaagaac tctgtagcac cgcctacata cctcgctctg ctaatcctgt 3060
   taccagtggc tgctgccagt ggcgataagt cgtgtcttac cgggttggac tcaagacgat 3120
   agttaccgga taaggcgcag cggtcgggct gaacgggggg ttcgtgcaca cagcccagct 3180
   tggagcgaac gacctacacc gaactgagat acctacagcg tgagctatga gaaagcgcca 3240
   cgcttcccga agggagaaag gcggacaggt atccggtaag cggcagggtc ggaacaggag 3300
   agcgcacgag ggagcttcca gggggaaacg cctggtatct ttatagtcct gtcgggtttc 3360
   gccacctctg acttgagcgt cgatttttgt gatgctcgtc aggggggcgg agcctatgga 3420
   aaaacgccag caacgcggcc tttttacggt tcctggcctt ttgctggcct tttgctcaca 3480
   tgttctttcc tgcgttatcc cctgattctg tggataaccg tattaccgcc tttgagtgag 3540
   ctgataccgc tcgccgcagc cgaacgaccg agcgcagcga gtcagtgagc gaggaagcgg 3600
   aagagcgcct gatgcggtat tttctcctta cgcatctgtg cggtatttca caccgcatat 3660
   atggtgcact ctcagtacaa tctgctctga tgccgcatag ttaagccagt atacactccg 3720
   ctatcgctac gtgactgggt catggctgcg ccccgacacc cgccaacacc cgctgacgcg 3780
   ccctgacggg cttgtctgct cccggcatcc gcttacagac aagctgtgac cgtctccggg 3840
   agctgcatgt gtcagaggtt ttcaccgtca tcaccgaaac gcgcgaggca gctgcggtaa 3900
   agctcatcag cgtggtcgtg aagcgattca cagatgtctg cctgttcatc cgcgtccagc 3960
   tcgttgagtt tctccagaag cgttaatgtc tggcttctga taaagcgggc catgttaagg 4020
   gcggtttttt cctgtttggt cactgatgcc tccgtgtaag ggggatttct gttcatgggg 4080
   gtaatgatac cgatgaaacg agagaggatg ctcacgatac gggttactga tgatgaacat 4140
   gcccggttac tggaacgttg tgagggtaaa caactggcgg tatggatgcg gcgggaccag 4200
   agaaaaatca ctcagggtca atgccagcgc ttcgttaata cagatgtagg tgttccacag 4260
   ggtagccagc agcatcctgc gatgcagatc cggaacataa tggtgcaggg cgctgacttc 4320
   cgcgtttcca gactttacga aacacggaaa ccgaagacca ttcatgttgt tgctcaggtc 4380
   gcagacgttt tgcagcagca gtcgcttcac gttcgctcgc gtatcggtga ttcattctgc 4440
   taaccagtaa ggcaaccccg ccagcctagc cgggtcctca acgacaggag cacgatcatg 4500
   cgcacccgtg gggccgccat gccggcgata atggcctgct tctcgccgaa acgtttggtg 4560
   gcgggaccag tgacgaaggc ttgagcgagg gcgtgcaaga ttccgaatac cgcaagcgac 4620
   aggccgatca tcgtcgcgct ccagcgaaag cggtcctcgc cgaaaatgac ccagagcgct 4680
   gccggcacct gtcctacgag ttgcatgata aagaagacag tcataagtgc ggcgacgata 4740
   gtcatgcccc gcgcccaccg gaaggagctg actgggttga aggctctcaa gggcatcggt 4800
   cgagatcccg gtgcctaatg agtgagctaa cttacattaa ttgcgttgcg ctcactgccc 4860
   gctttccagt cgggaaacct gtcgtgccag ctgcattaat gaatcggcca acgcgcgggg 4920
   agaggcggtt tgcgtattgg gcgccagggt ggtttttctt ttcaccagtg agacgggcaa 4980
   cagctgattg cccttcaccg cctggccctg agagagttgc agcaagcggt ccacgctggt 5040
   ttgccccagc aggcgaaaat cctgtttgat ggtggttaac ggcgggatat aacatgagct 5100
   gtcttcggta tcgtcgtatc ccactaccga gatatccgca ccaacgcgca gcccggactc 5160
   ggtaatggcg cgcattgcgc ccagcgccat ctgatcgttg gcaaccagca tcgcagtggg 5220
   aacgatgccc tcattcagca tttgcatggt ttgttgaaaa ccggacatgg cactccagtc 5280
   gccttcccgt tccgctatcg gctgaatttg attgcgagtg agatatttat gccagccagc 5340
   cagacgcaga cgcgccgaga cagaacttaa tgggcccgct aacagcgcga tttgctggtg 5400
   acccaatgcg accagatgct ccacgcccag tcgcgtaccg tcttcatggg agaaaataat 5460
   actgttgatg ggtgtctggt cagagacatc aagaaataac gccggaacat tagtgcaggc 5520
   agcttccaca gcaatggcat cctggtcatc cagcggatag ttaatgatca gcccactgac 5580
   gcgttgcgcg agaagattgt gcaccgccgc tttacaggct tcgacgccgc ttcgttctac 5640
   catcgacacc accacgctgg cacccagttg atcggcgcga gatttaatcg ccgcgacaat 5700
   ttgcgacggc gcgtgcaggg ccagactgga ggtggcaacg ccaatcagca acgactgttt 5760
   gcccgccagt tgttgtgcca cgcggttggg aatgtaattc agctccgcca tcgccgcttc 5820
   cactttttcc cgcgttttcg cagaaacgtg gctggcctgg ttcaccacgc gggaaacggt 5880
   ctgataagag acaccggcat actctgcgac atcgtataac gttactggtt tcacattcac 5940
   caccctgaat tgactctctt ccgggcgcta tcatgccata ccgcgaaagg ttttgcgcca 6000
   ttcgatggtg tccgggatct cgacgctctc ccttatgcga ctcctgcatt aggaagcagc 6060
   ccagtagtag gttgaggccg ttgagcaccg ccgccgcaag gaatggtgca tgcaaggaga 6120
   tggcgcccaa cagtcccccg gccacggggc ctgccaccat acccacgccg aaacaagcgc 6180
   tcatgagccc gaagtggcga gcccgatctt ccccatcggt gatgtcggcg atataggcgc 6240
   cagcaaccgc acctgtggcg ccggtgatgc cggccacgat gcgtccggcg tagaggatcg 6300
   agatctcgat cccgcgaaat taatacgact cactata 6337
<210> 9
   <211> 6337
   <212> DNA
   <213> artificial
<220>
   <223> pET24d-Mup44 (opt.)
<400> 9
   ggggaattgt gagcggataa caattcccct ctagaaataa ttttgtttaa ctttaagaag      60
   gagatatacc atggaaccag gtcaacctag agaacctcag gaacctaggg aaccaggtcc     120
   aggagctgaa acagctgctg ctccagtgtg ggaggaagcg aaaatcttct acgacaatct     180
   ggcacccaag aagaaaccca agagtccgaa accccagaat gccgtgacaa ttgccgtgtc     240
   aagcagggct ctgttccgaa tggacgaaga acagcagatc tacaccgagc aaggcgtgga     300
   agaatacgtt cgctatcagc tcgaacacga aaatgaaccg ttcagccctg gacccgcatt     360
   tccctttgtt aaagccctgg aagcggtcaa caggagactg agggagcttt accctgattc     420
   tgaggacgtc tttgacatcg tgctgatgac aaacaaccat gctcaggtgg gagttcgcct     480
   gatcaactcc atcaaccact atgacctgtt tatagagcgc ttttgcatga ccggtggaaa     540
   ctccccaatt tgctacctca aagcctatca caccaatctg tacctttccg ctgatgccga    600
   gaaggtccga gaggcaatcg atgagggtat tgcagcagct acgatcttca gtccatccag     660
   agatgtcgtg gtgtctcaaa gccagctgag agtggcgttt gatggagatg ccgtactctt     720
   cagcgacgag tcagagcgta ttgtgaaggc acatgggctc gaccggttct ttgagcatga     780
   gaaggcccac gagaataagc ctcttgccca aggacctctg aaagggtttc tggaggcact     840
   gggcaggctg cagaagaagt tctatagcaa aggtttgcgc ttggagtgtc caatacggac     900
   ctatctcgtc acagctagat ctgctgcaag cagtggagcc cgagctctga aaacgttgcg     960
   ttcatggggc cttgagactg acgaggctct ctttctggct ggagctccca aaggtcctct    1020
   gttggagaag atacggcctc acattttctt cgatgaccag atgttccacg ttgcaggtgc    1080
   tcaggagatg ggcaccgtag ccgctcatgt gccatacgga gtagcgcaaa ctcccaggag    1140
   aactgcacca gccaagcagg caccctctgc acagctcgag caccaccacc accaccactg    1200
   agatccggct gctaacaaag cccgaaagga agctgagttg gctgctgcca ccgctgagca    1260
   ataactagca taaccccttg gggcctctaa acgggtcttg aggggttttt tgctgaaagg    1320
   aggaactata tccggattgg cgaatgggac gcgccctgta gcggcgcatt aagcgcggcg    1380
   ggtgtggtgg ttacgcgcag cgtgaccgct acacttgcca gcgccctagc gcccgctcct    1440
   ttcgctttct tcccttcctt tctcgccacg ttcgccggct ttccccgtca agctctaaat    1500
   cgggggctcc ctttagggtt ccgatttagt gctttacggc acctcgaccc caaaaaactt    1560
   gattagggtg atggttcacg tagtgggcca tcgccctgat agacggtttt tcgccctttg    1620
   acgttggagt ccacgttctt taatagtgga ctcttgttcc aaactggaac aacactcaac    1680
   cctatctcgg tctattcttt tgatttataa gggattttgc cgatttcggc ctattggtta    1740
   aaaaatgagc tgatttaaca aaaatttaac gcgaatttta acaaaatatt aacgtttaca    1800
   atttcaggtg gcacttttcg gggaaatgtg cgcggaaccc ctatttgttt atttttctaa    1860
   atacattcaa atatgtatcc gctcatgaat taattcttag aaaaactcat cgagcatcaa    1920
   atgaaactgc aatttattca tatcaggatt atcaatacca tatttttgaa aaagccgttt    1980
   ctgtaatgaa ggagaaaact caccgaggca gttccatagg atggcaagat cctggtatcg    2040
   gtctgcgatt ccgactcgtc caacatcaat acaacctatt aatttcccct cgtcaaaaat    2100
   aaggttatca agtgagaaat caccatgagt gacgactgaa tccggtgaga atggcaaaag    2160
   tttatgcatt tctttccaga cttgttcaac aggccagcca ttacgctcgt catcaaaatc    2220
   actcgcatca accaaaccgt tattcattcg tgattgcgcc tgagcgagac gaaatacgcg    2280
   atcgctgtta aaaggacaat tacaaacagg aatcgaatgc aaccggcgca ggaacactgc    2340
   cagcgcatca acaatatttt cacctgaatc aggatattct tctaatacct ggaatgctgt    2400
   tttcccgggg atcgcagtgg tgagtaacca tgcatcatca ggagtacgga taaaatgctt    2460
   gatggtcgga agaggcataa attccgtcag ccagtttagt ctgaccatct catctgtaac    2520
   atcattggca acgctacctt tgccatgttt cagaaacaac tctggcgcat cgggcttccc    2580
   atacaatcga tagattgtcg cacctgattg cccgacatta tcgcgagccc atttataccc    2640
   atataaatca gcatccatgt tggaatttaa tcgcggccta gagcaagacg tttcccgttg    2700
   aatatggctc ataacacccc ttgtattact gtttatgtaa gcagacagtt ttattgttca    2760
   tgaccaaaat cccttaacgt gagttttcgt tccactgagc gtcagacccc gtagaaaaga    2820
   tcaaaggatc ttcttgagat cctttttttc tgcgcgtaat ctgctgcttg caaacaaaaa    2880
   aaccaccgct accagcggtg gtttgtttgc cggatcaaga gctaccaact ctttttccga    2940
   aggtaactgg cttcagcaga gcgcagatac caaatactgt ccttctagtg tagccgtagt    3000
   taggccacca cttcaagaac tctgtagcac cgcctacata cctcgctctg ctaatcctgt    3060
   taccagtggc tgctgccagt ggcgataagt cgtgtcttac cgggttggac tcaagacgat    3120
   agttaccgga taaggcgcag cggtcgggct gaacgggggg ttcgtgcaca cagcccagct    3180
   tggagcgaac gacctacacc gaactgagat acctacagcg tgagctatga gaaagcgcca    3240
   cgcttcccga agggagaaag gcggacaggt atccggtaag cggcagggtc ggaacaggag    3300
   agcgcacgag ggagcttcca gggggaaacg cctggtatct ttatagtcct gtcgggtttc    3360
   gccacctctg acttgagcgt cgatttttgt gatgctcgtc aggggggcgg agcctatgga 3420
   aaaacgccag caacgcggcc tttttacggt tcctggcctt ttgctggcct tttgctcaca 3480
   tgttctttcc tgcgttatcc cctgattctg tggataaccg tattaccgcc tttgagtgag 3540
   ctgataccgc tcgccgcagc cgaacgaccg agcgcagcga gtcagtgagc gaggaagcgg 3600
   aagagcgcct gatgcggtat tttctcctta cgcatctgtg cggtatttca caccgcatat 3660
   atggtgcact ctcagtacaa tctgctctga tgccgcatag ttaagccagt atacactccg 3720
   ctatcgctac gtgactgggt catggctgcg ccccgacacc cgccaacacc cgctgacgcg 3780
   ccctgacggg cttgtctgct cccggcatcc gcttacagac aagctgtgac cgtctccggg 3840
   agctgcatgt gtcagaggtt ttcaccgtca tcaccgaaac gcgcgaggca gctgcggtaa 3900
   agctcatcag cgtggtcgtg aagcgattca cagatgtctg cctgttcatc cgcgtccagc 3960
   tcgttgagtt tctccagaag cgttaatgtc tggcttctga taaagcgggc catgttaagg 4020
   gcggtttttt cctgtttggt cactgatgcc tccgtgtaag ggggatttct gttcatgggg 4080
   gtaatgatac cgatgaaacg agagaggatg ctcacgatac gggttactga tgatgaacat 4140
   gcccggttac tggaacgttg tgagggtaaa caactggcgg tatggatgcg gcgggaccag 4200
   agaaaaatca ctcagggtca atgccagcgc ttcgttaata cagatgtagg tgttccacag 4260
   ggtagccagc agcatcctgc gatgcagatc cggaacataa tggtgcaggg cgctgacttc 4320
   cgcgtttcca gactttacga aacacggaaa ccgaagacca ttcatgttgt tgctcaggtc 4380
   gcagacgttt tgcagcagca gtcgcttcac gttcgctcgc gtatcggtga ttcattctgc 4440
   taaccagtaa ggcaaccccg ccagcctagc cgggtcctca acgacaggag cacgatcatg 4500
   cgcacccgtg gggccgccat gccggcgata atggcctgct tctcgccgaa acgtttggtg 4560
   gcgggaccag tgacgaaggc ttgagcgagg gcgtgcaaga ttccgaatac cgcaagcgac 4620
   aggccgatca tcgtcgcgct ccagcgaaag cggtcctcgc cgaaaatgac ccagagcgct 4680
   gccggcacct gtcctacgag ttgcatgata aagaagacag tcataagtgc ggcgacgata 4740
   gtcatgcccc gcgcccaccg gaaggagctg actgggttga aggctctcaa gggcatcggt 4800
   cgagatcccg gtgcctaatg agtgagctaa cttacattaa ttgcgttgcg ctcactgccc 4860
   gctttccagt cgggaaacct gtcgtgccag ctgcattaat gaatcggcca acgcgcgggg 4920
   agaggcggtt tgcgtattgg gcgccagggt ggtttttctt ttcaccagtg agacgggcaa 4980
   cagctgattg cccttcaccg cctggccctg agagagttgc agcaagcggt ccacgctggt 5040
   ttgccccagc aggcgaaaat cctgtttgat ggtggttaac ggcgggatat aacatgagct 5100
   gtcttcggta tcgtcgtatc ccactaccga gatatccgca ccaacgcgca gcccggactc 5160
   ggtaatggcg cgcattgcgc ccagcgccat ctgatcgttg gcaaccagca tcgcagtggg 5220
   aacgatgccc tcattcagca tttgcatggt ttgttgaaaa ccggacatgg cactccagtc 5280
   gccttcccgt tccgctatcg gctgaatttg attgcgagtg agatatttat gccagccagc 5340
   cagacgcaga cgcgccgaga cagaacttaa tgggcccgct aacagcgcga tttgctggtg 5400
   acccaatgcg accagatgct ccacgcccag tcgcgtaccg tcttcatggg agaaaataat    5460
   actgttgatg ggtgtctggt cagagacatc aagaaataac gccggaacat tagtgcaggc    5520
   agcttccaca gcaatggcat cctggtcatc cagcggatag ttaatgatca gcccactgac    5580
   gcgttgcgcg agaagattgt gcaccgccgc tttacaggct tcgacgccgc ttcgttctac    5640
   catcgacacc accacgctgg cacccagttg atcggcgcga gatttaatcg ccgcgacaat    5700
   ttgcgacggc gcgtgcaggg ccagactgga ggtggcaacg ccaatcagca acgactgttt    5760
   gcccgccagt tgttgtgcca cgcggttggg aatgtaattc agctccgcca tcgccgcttc    5820
   cactttttcc cgcgttttcg cagaaacgtg gctggcctgg ttcaccacgc gggaaacggt    5880
   ctgataagag acaccggcat actctgcgac atcgtataac gttactggtt tcacattcac    5940
   caccctgaat tgactctctt ccgggcgcta tcatgccata ccgcgaaagg ttttgcgcca    6000
   ttcgatggtg tccgggatct cgacgctctc ccttatgcga ctcctgcatt aggaagcagc    6060
   ccagtagtag gttgaggccg ttgagcaccg ccgccgcaag gaatggtgca tgcaaggaga    6120
   tggcgcccaa cagtcccccg gccacggggc ctgccaccat acccacgccg aaacaagcgc    6180
   tcatgagccc gaagtggcga gcccgatctt ccccatcggt gatgtcggcg atataggcgc    6240
   cagcaaccgc acctgtggcg ccggtgatgc cggccacgat gcgtccggcg tagaggatcg    6300
   agatctcgat cccgcgaaat taatacgact cactata                             6337
<210> 10
   <211> 6337
   <212> DNA
   <213> artificial
<220>
   <223> pET24d-Mup44 (opt.) [D211A]
<400> 10
   ggggaattgt gagcggataa caattcccct ctagaaataa ttttgtttaa ctttaagaag     60
   gagatatacc atggaaccag gtcaacctag agaacctcag gaacctaggg aaccaggtcc    120
   aggagctgaa acagctgctg ctccagtgtg ggaggaagcg aaaatcttct acgacaatct    180
   ggcacccaag aagaaaccca agagtccgaa accccagaat gccgtgacaa ttgccgtgtc    240
   aagcagggct ctgttccgaa tggacgaaga acagcagatc tacaccgagc aaggcgtgga    300
   agaatacgtt cgctatcagc tcgaacacga aaatgaaccg ttcagccctg gacccgcatt    360
   tccctttgtt aaagccctgg aagcggtcaa caggagactg agggagcttt accctgattc    420
   tgaggacgtc tttgacatcg tgctgatgac aaacaaccat gctcaggtgg gagttcgcct    480
   gatcaactcc atcaaccact atgacctgtt tatagagcgc ttttgcatga ccggtggaaa    540
   ctccccaatt tgctacctca aagcctatca caccaatctg tacctttccg ctgatgccga    600
   gaaggtccga gaggcaatcg atgagggtat tgcagcagct acgatcttca gtccatccag    660
   agatgtcgtg gtgtctcaaa gccagctgag agtggcgttt gcaggagatg ccgtactctt    720
   cagcgacgag tcagagcgta ttgtgaaggc acatgggctc gaccggttct ttgagcatga    780
   gaaggcccac gagaataagc ctcttgccca aggacctctg aaagggtttc tggaggcact 840
   gggcaggctg cagaagaagt tctatagcaa aggtttgcgc ttggagtgtc caatacggac 900
   ctatctcgtc acagctagat ctgctgcaag cagtggagcc cgagctctga aaacgttgcg 960
   ttcatggggc cttgagactg acgaggctct ctttctggct ggagctccca aaggtcctct 1020
   gttggagaag atacggcctc acattttctt cgatgaccag atgttccacg ttgcaggtgc 1080
   tcaggagatg ggcaccgtag ccgctcatgt gccatacgga gtagcgcaaa ctcccaggag 1140
   aactgcacca gccaagcagg caccctctgc acagctcgag caccaccacc accaccactg 1200
   agatccggct gctaacaaag cccgaaagga agctgagttg gctgctgcca ccgctgagca 1260
   ataactagca taaccccttg gggcctctaa acgggtcttg aggggttttt tgctgaaagg 1320
   aggaactata tccggattgg cgaatgggac gcgccctgta gcggcgcatt aagcgcggcg 1380
   ggtgtggtgg ttacgcgcag cgtgaccgct acacttgcca gcgccctagc gcccgctcct 1440
   ttcgctttct tcccttcctt tctcgccacg ttcgccggct ttccccgtca agctctaaat 1500
   cgggggctcc ctttagggtt ccgatttagt gctttacggc acctcgaccc caaaaaactt 1560
   gattagggtg atggttcacg tagtgggcca tcgccctgat agacggtttt tcgccctttg 1620
   acgttggagt ccacgttctt taatagtgga ctcttgttcc aaactggaac aacactcaac 1680
   cctatctcgg tctattcttt tgatttataa gggattttgc cgatttcggc ctattggtta 1740
   aaaaatgagc tgatttaaca aaaatttaac gcgaatttta acaaaatatt aacgtttaca 1800
   atttcaggtg gcacttttcg gggaaatgtg cgcggaaccc ctatttgttt atttttctaa 1860
   atacattcaa atatgtatcc gctcatgaat taattcttag aaaaactcat cgagcatcaa 1920
   atgaaactgc aatttattca tatcaggatt atcaatacca tatttttgaa aaagccgttt 1980
   ctgtaatgaa ggagaaaact caccgaggca gttccatagg atggcaagat cctggtatcg 2040
   gtctgcgatt ccgactcgtc caacatcaat acaacctatt aatttcccct cgtcaaaaat 2100
   aaggttatca agtgagaaat caccatgagt gacgactgaa tccggtgaga atggcaaaag 2160
   tttatgcatt tctttccaga cttgttcaac aggccagcca ttacgctcgt catcaaaatc 2220
   actcgcatca accaaaccgt tattcattcg tgattgcgcc tgagcgagac gaaatacgcg 2280
   atcgctgtta aaaggacaat tacaaacagg aatcgaatgc aaccggcgca ggaacactgc 2340
   cagcgcatca acaatatttt cacctgaatc aggatattct tctaatacct ggaatgctgt 2400
   tttcccgggg atcgcagtgg tgagtaacca tgcatcatca ggagtacgga taaaatgctt 2460
   gatggtcgga agaggcataa attccgtcag ccagtttagt ctgaccatct catctgtaac 2520
   atcattggca acgctacctt tgccatgttt cagaaacaac tctggcgcat cgggcttccc 2580
   atacaatcga tagattgtcg cacctgattg cccgacatta tcgcgagccc atttataccc 2640
   atataaatca gcatccatgt tggaatttaa tcgcggccta gagcaagacg tttcccgttg 2700
   aatatggctc ataacacccc ttgtattact gtttatgtaa gcagacagtt ttattgttca 2760
   tgaccaaaat cccttaacgt gagttttcgt tccactgagc gtcagacccc gtagaaaaga 2820
   tcaaaggatc ttcttgagat cctttttttc tgcgcgtaat ctgctgcttg caaacaaaaa    2880
   aaccaccgct accagcggtg gtttgtttgc cggatcaaga gctaccaact ctttttccga    2940
   aggtaactgg cttcagcaga gcgcagatac caaatactgt ccttctagtg tagccgtagt    3000
   taggccacca cttcaagaac tctgtagcac cgcctacata cctcgctctg ctaatcctgt    3060
   taccagtggc tgctgccagt ggcgataagt cgtgtcttac cgggttggac tcaagacgat    3120
   agttaccgga taaggcgcag cggtcgggct gaacgggggg ttcgtgcaca cagcccagct    3180
   tggagcgaac gacctacacc gaactgagat acctacagcg tgagctatga gaaagcgcca    3240
   cgcttcccga agggagaaag gcggacaggt atccggtaag cggcagggtc ggaacaggag    3300
   agcgcacgag ggagcttcca gggggaaacg cctggtatct ttatagtcct gtcgggtttc    3360
   gccacctctg acttgagcgt cgatttttgt gatgctcgtc aggggggcgg agcctatgga    3420
   aaaacgccag caacgcggcc tttttacggt tcctggcctt ttgctggcct tttgctcaca    3480
   tgttctttcc tgcgttatcc cctgattctg tggataaccg tattaccgcc tttgagtgag    3540
   ctgataccgc tcgccgcagc cgaacgaccg agcgcagcga gtcagtgagc gaggaagcgg    3600
   aagagcgcct gatgcggtat tttctcctta cgcatctgtg cggtatttca caccgcatat    3660
   atggtgcact ctcagtacaa tctgctctga tgccgcatag ttaagccagt atacactccg    3720
   ctatcgctac gtgactgggt catggctgcg ccccgacacc cgccaacacc cgctgacgcg    3780
   ccctgacggg cttgtctgct cccggcatcc gcttacagac aagctgtgac cgtctccggg    3840
   agctgcatgt gtcagaggtt ttcaccgtca tcaccgaaac gcgcgaggca gctgcggtaa    3900
   agctcatcag cgtggtcgtg aagcgattca cagatgtctg cctgttcatc cgcgtccagc    3960
   tcgttgagtt tctccagaag cgttaatgtc tggcttctga taaagcgggc catgttaagg    4020
   gcggtttttt cctgtttggt cactgatgcc tccgtgtaag ggggatttct gttcatgggg    4080
   gtaatgatac cgatgaaacg agagaggatg ctcacgatac gggttactga tgatgaacat    4140
   gcccggttac tggaacgttg tgagggtaaa caactggcgg tatggatgcg gcgggaccag    4200
   agaaaaatca ctcagggtca atgccagcgc ttcgttaata cagatgtagg tgttccacag    4260
   ggtagccagc agcatcctgc gatgcagatc cggaacataa tggtgcaggg cgctgacttc    4320
   cgcgtttcca gactttacga aacacggaaa ccgaagacca ttcatgttgt tgctcaggtc    4380
   gcagacgttt tgcagcagca gtcgcttcac gttcgctcgc gtatcggtga ttcattctgc    4440
   taaccagtaa ggcaaccccg ccagcctagc cgggtcctca acgacaggag cacgatcatg    4500
   cgcacccgtg gggccgccat gccggcgata atggcctgct tctcgccgaa acgtttggtg    4560
   gcgggaccag tgacgaaggc ttgagcgagg gcgtgcaaga ttccgaatac cgcaagcgac    4620
   aggccgatca tcgtcgcgct ccagcgaaag cggtcctcgc cgaaaatgac ccagagcgct    4680
   gccggcacct gtcctacgag ttgcatgata aagaagacag tcataagtgc ggcgacgata    4740
   gtcatgcccc gcgcccaccg gaaggagctg actgggttga aggctctcaa gggcatcggt    4800
   cgagatcccg gtgcctaatg agtgagctaa cttacattaa ttgcgttgcg ctcactgccc    4860
   gctttccagt cgggaaacct gtcgtgccag ctgcattaat gaatcggcca acgcgcgggg 4920
   agaggcggtt tgcgtattgg gcgccagggt ggtttttctt ttcaccagtg agacgggcaa 4980
   cagctgattg cccttcaccg cctggccctg agagagttgc agcaagcggt ccacgctggt 5040
   ttgccccagc aggcgaaaat cctgtttgat ggtggttaac ggcgggatat aacatgagct 5100
   gtcttcggta tcgtcgtatc ccactaccga gatatccgca ccaacgcgca gcccggactc 5160
   ggtaatggcg cgcattgcgc ccagcgccat ctgatcgttg gcaaccagca tcgcagtggg 5220
   aacgatgccc tcattcagca tttgcatggt ttgttgaaaa ccggacatgg cactccagtc 5280
   gccttcccgt tccgctatcg gctgaatttg attgcgagtg agatatttat gccagccagc 5340
   cagacgcaga cgcgccgaga cagaacttaa tgggcccgct aacagcgcga tttgctggtg 5400
   acccaatgcg accagatgct ccacgcccag tcgcgtaccg tcttcatggg agaaaataat 5460
   actgttgatg ggtgtctggt cagagacatc aagaaataac gccggaacat tagtgcaggc 5520
   agcttccaca gcaatggcat cctggtcatc cagcggatag ttaatgatca gcccactgac 5580
   gcgttgcgcg agaagattgt gcaccgccgc tttacaggct tcgacgccgc ttcgttctac 5640
   catcgacacc accacgctgg cacccagttg atcggcgcga gatttaatcg ccgcgacaat 5700
   ttgcgacggc gcgtgcaggg ccagactgga ggtggcaacg ccaatcagca acgactgttt 5760
   gcccgccagt tgttgtgcca cgcggttggg aatgtaattc agctccgcca tcgccgcttc 5820
   cactttttcc cgcgttttcg cagaaacgtg gctggcctgg ttcaccacgc gggaaacggt 5880
   ctgataagag acaccggcat actctgcgac atcgtataac gttactggtt tcacattcac 5940
   caccctgaat tgactctctt ccgggcgcta tcatgccata ccgcgaaagg ttttgcgcca 6000
   ttcgatggtg tccgggatct cgacgctctc ccttatgcga ctcctgcatt aggaagcagc 6060
   ccagtagtag gttgaggccg ttgagcaccg ccgccgcaag gaatggtgca tgcaaggaga 6120
   tggcgcccaa cagtcccccg gccacggggc ctgccaccat acccacgccg aaacaagcgc 6180
   tcatgagccc gaagtggcga gcccgatctt ccccatcggt gatgtcggcg atataggcgc 6240
   cagcaaccgc acctgtggcg ccggtgatgc cggccacgat gcgtccggcg tagaggatcg 6300
   agatctcgat cccgcgaaat taatacgact cactata 6337
<210> 11
   <211> 6718
   <212> DNA
   <213> artificial
<220>
   <223> pTriEX-Mup44
<400> 11
   ggggaattgt gagcggataa caattccccg gagttaatcc gggaccttta attcaaccca      60
   acacaatata ttatagttaa ataagaatta ttatcaaatc atttgtatat taattaaaat     120
   actatactgt aaattacatt ttatttacaa tcaaaggaga tataccatgg aacctgggca     180
   gccccgggag ccccaggagc cccgcgagcc cgggccagga gcggagaccg ctgcggcccc     240
   ggtctgggag gaagccaaga ttttctacga caacctcgcg cccaagaaga aacccaaatc 300
   gcccaagcct cagaatgcag tcaccatcgc tgtgtcctcc cgagccttgt ttcgcatgga 360
   cgaggagcag cagatctaca cggagcaggg cgtggaggag tacgtgcgct accagctgga 420
   acatgagaac gaacccttca gtcccgggcc agccttccct tttgtgaagg ctctggaggc 480
   cgtgaacagg cggctgcggg agctgtaccc tgatagtgag gacgtcttcg acatcgtcct 540
   catgactaac aaccatgctc aagtgggtgt ccgcctcatc aacagtatca accactatga 600
   cctgttcatc gagaggttct gcatgacagg tgggaacagc ccgatctgct acctcaaggc 660
   ctatcacacc aacctctact tgtcagccga tgcggaaaaa gtgcgagaag ccattgatga 720
   ggggatcgca gctgccacca tcttcagccc cagcagggat gtggttgtgt cccagagtca 780
   gctgcgcgtg gccttcgatg gggacgccgt gctcttctcg gacgagtcgg agcgcatcgt 840
   caaggcccac gggctggacc gattcttcga gcatgagaag gcccacgaga acaaacctct 900
   ggctcagggc cccttaaagg gctttctgga ggcactgggt aggttgcaga agaagttcta 960
   ctccaaaggc ctgcggctgg agtgcccaat tcgtacctac ttggtgacag cacgcagtgc 1020
   agccagttcc ggggcccggg ctctcaagac cctgcgcagc tggggcctgg agacagatga 1080
   agccttgttc cttgctggag cgcccaaggg ccctctcctt gagaagatcc gcccacacat 1140
   cttctttgat gaccagatgt tccatgtggc tggggctcag gagatgggca ctgtggccgc 1200
   ccatgtgcct tatggtgtgg cacagacacc ccggcggact gcacctgcaa agcaggcccc 1260
   atctgcacag ctcgagcacc accatcacca tcaccatcac taagtgatta acctcaggtg 1320
   caggctgcct atcagaaggt ggtggctggt gtggccaatg ccctggctca caaataccac 1380
   tgagatcgat ctttttccct ctgccaaaaa ttatggggac atcatgaagc cccttgagca 1440
   tctgacttct ggctaataaa ggaaatttat tttcattgca atagtgtgtt ggaatttttt 1500
   gtgtctctca ctcggaagga catatgggag ggcaaatcat ttaaaacatc agaatgagta 1560
   tttggtttag agtttggcaa catatgccca tatgtaacta gcataacccc ttggggcctc 1620
   taaacgggtc ttgaggggtt ttttgctgaa agcatgcgga ggaaattctc cttgaagttt 1680
   ccctggtgtt caaagtaaag gagtttgcac cagacgcacc tctgttcact ggtccggcgt 1740
   attaaaacac gatacattgt tattagtaca tttattaagc gctagattct gtgcgttgtt 1800
   gatttacaga caattgttgt acgtatttta ataattcatt aaatttataa tctttagggt 1860
   ggtatgttag agcgaaaatc aaatgatttt cagcgtcttt atatctgaat ttaaatatta 1920
   aatcctcaat agatttgtaa aataggtttc gattagtttc aaacaagggt tgtttttccg 1980
   aaccgatggc tggactatct aatggatttt cgctcaacgc cacaaaactt gccaaatctt 2040
   gtagcagcaa tctagctttg tcgatattcg tttgtgtttt gttttgtaat aaaggttcga 2100
   cgtcgttcaa aatattatgc gcttttgtat ttctttcatc actgtcgtta gtgtacaatt 2160
   gactcgacgt aaacacgtta aatagagctt ggacatattt aacatcgggc gtgttagctt 2220
   tattaggccg attatcgtcg tcgtcccaac cctcgtcgtt agaagttgct tccgaagacg 2280
   attttgccat agccacacga cgcctattaa ttgtgtcggc taacacgtcc gcgatcaaat 2340
   ttgtagttga gctttttgga attatttctg attgcgggcg tttttgggcg ggtttcaatc 2400
   taactgtgcc cgattttaat tcagacaaca cgttagaaag cgatggtgca ggcggtggta 2460
   acatttcaga cggcaaatct actaatggcg gcggtggtgg agctgatgat aaatctacca 2520
   tcggtggagg cgcaggcggg gctggcggcg gaggcggagg cggaggtggt ggcggtgatg 2580
   cagacggcgg tttaggctca aatgtctctt taggcaacac agtcggcacc tcaactattg 2640
   tactggtttc gggcgccgtt tttggtttga ccggtctgag acgagtgcga tttttttcgt 2700
   ttctaatagc ttccaacaat tgttgtctgt cgtctaaagg tgcagcgggt tgaggttccg 2760
   tcggcattgg tggagcgggc ggcaattcag acatcgatgg tggtggtggt ggtggaggcg 2820
   ctggaatgtt aggcacggga gaaggtggtg gcggcggtgc cgccggtata atttgttctg 2880
   gtttagtttg ttcgcgcacg attgtgggca ccggcgcagg cgccgctggc tgcacaacgg 2940
   aaggtcgtct gcttcgaggc agcgcttggg gtggtggcaa ttcaatatta taattggaat 3000
   acaaatcgta aaaatctgct ataagcattg taatttcgct atcgtttacc gtgccgatat 3060
   ttaacaaccg ctcaatgtaa gcaattgtat tgtaaagaga ttgtctcaag ctcggaacgc 3120
   tgcgctcggt cgttcggctg cggcgagcgg tatcagctca ctcaaaggcg gtaatacggt 3180
   tatccacaga atcaggggat aacgcaggaa agaacatgtg agcaaaaggc cagcaaaagg 3240
   ccaggaaccg taaaaaggcc gcgttgctgg cgtttttcca taggctccgc ccccctgacg 3300
   agcatcacaa aaatcgacgc tcaagtcaga ggtggcgaaa cccgacagga ctataaagat 3360
   accaggcgtt tccccctgga agctccctcg tgcgctctcc tgttccgacc ctgccgctta 3420
   ccggatacct gtccgccttt ctcccttcgg gaagcgtggc gctttctcaa tgctcacgct 3480
   gtaggtatct cagttcggtg taggtcgttc gctccaagct gggctgtgtg cacgaacccc 3540
   ccgttcagcc cgaccgctgc gccttatccg gtaactatcg tcttgagtcc aacccggtaa 3600
   gacacgactt atcgccactg gcagcagcca ctggtaacag gattagcaga gcgaggtatg 3660
   taggcggtgc tacagagttc ttgaagtggt ggcctaacta cggctacact agaaggacag 3720
   tatttggtat ctgcgctctg ctgaagccag ttaccttcgg aaaaagagtt ggtagctctt 3780
   gatccggcaa acaaaccacc gctggtagcg gtggtttttt tgtttgcaag cagcagatta 3840
   cgcgcagaaa aaaaggatct caagaagatc ctttgttacc aatgcttaat cagtgaggca 3900
   cctatctcag cgatctgtct atttcgttca tccatagttg cctgactccc cgtcgtgtag 3960
   ataactacga tacgggaggg cttaccatct ggccccagtg ctgcaatgat accgcgagac 4020
   ccacgctcac cggctccaga tttatcagca ataaaccagc cagccggaag ggccgagcgc 4080
   agaagtggtc ctgcaacttt atccgcctcc atccagtcta ttaattgttg ccgggaagct 4140
   agagtaagta gttcgccagt taatagtttg cgcaacgttg ttgccattgc tacaggcatc 4200
   gtggtgtcac gctcgtcgtt tggtatggct tcattcagct ccggttccca acgatcaagg 4260
   cgagttacat gatcccccat gttgtgcaaa aaagcggtta gctccttcgg tcctccgatc 4320
   gttgtcagaa gtaagttggc cgcagtgtta tcactcatgg ttatggcagc actgcataat 4380
   tctcttactg tcatgccatc cgtaagatgc ttttctgtga ctggtgagta ctcaaccaag 4440
   tcattctgag aatagtgtat gcggcgaccg agttgctctt gcccggcgtc aatacgggat 4500
   aataccgcgc cacatagcag aactttaaaa gtgctcatca ttggaaaacg ttcttcgggg 4560
   cgaaaactct caaggatctt accgctgttg agatccagtt cgatgtaacc cactcgtgca 4620
   cccaactgat cttcagcatc ttttactttc accagcgttt ctgggtgagc aaaaacagga 4680
   aggcaaaatg ccgcaaaaaa gggaataagg gcgacacgga aatgttgaat actcatactc 4740
   ttcctttttc aatattattg aagcatttat cagggttatt gtctcatgtc cgcgcgtttc 4800
   ctgcatcttt taatcaaatc ccaagatgtg tataaaccac caaactgcca aaaaatgaaa 4860
   actgtcgaca agctctgtcc gtttgctggc aactgcaagg gtctcaatcc tatttgtaat 4920
   tattgaataa taaaacaatt ataaatgtca aatttgtttt ttattaacga tacaaaccaa 4980
   acgcaacaag aacatttgta gtattatcta taattgaaaa cgcgtagtta taatcgctga 5040
   ggtaatattt aaaatcattt tcaaatgatt cacagttaat ttgcgacaat ataattttat 5100
   tttcacataa actagacgcc ttgtcgtctt cttcttcgta ttccttctct tttcattttt 5160
   tctcttcata aaaattaaca tagttattat cgtatccata tatgtatcta tcgtatagag 5220
   taaatttttt gttgtcataa atatatatgt cttttttaat ggggtgtata gtaccgctgc 5280
   gcatagtttt tctgtaattt acaacagtgc tattttctgg tagttcttcg gagtgtgttg 5340
   ctttaattat taaatttata taatcaatga atttgggatc gtcggttttg tacaatatgt 5400
   tgccggcata gtacgcagct tcttctagtt caattacacc attttttagc agcaccggat 5460
   taacataact ttccaaaatg ttgtacgaac cgttaaacaa aaacagttca cctccctttt 5520
   ctatactatt gtctgcgagc agttgtttgt tgttaaaaat aacagccatt gtaatgagac 5580
   gcacaaacta atatcacaaa ctggaaatgt ctatcaatat atagttgctc tagttattaa 5640
   tagtaatcaa ttacggggtc attagttcat agcccatata tggagttccg cgttacataa 5700
   cttacggtaa atggcccgcc tggctgaccg cccaacgacc cccgcccatt gacgtcaata 5760
   atgacgtatg ttcccatagt aacgccaata gggactttcc attgacgtca atgggtggac 5820
   tatttacggt aaactgccca cttggcagta catcaagtgt atcatatgcc aagtacgccc 5880
   cctattgacg tcaatgacgg taaatggccc gcctggcatt atgcccagta catgacctta 5940
   tgggactttc ctacttggca gtacatctac gtattagtca tcgctattac catgcatggt 6000
   cgaggtgagc cccacgttct gcttcactct ccccatctcc cccccctccc cacccccaat 6060
   tttgtattta tttatttttt aattattttg tgcagcgatg ggggcggggg gggggggggg 6120
   gcgcgcgcca ggcggggcgg ggcggggcga ggggcggggc ggggcgaggc ggagaggtgc 6180
   ggcggcagcc aatcagagcg gcgcgctccg aaagtttcct tttatggcga ggcggcggcg 6240
   gcggcggccc tataaaaagc gaagcgcgcg gcgggcggga gtcgctgcga cgctgccttc 6300
   gccccgtgcc ccgctccgcc gccgcctcgc gccgcccgcc ccggctctga ctgaccgcgt 6360
   tactcccaca ggtgagcggg cgggacggcc cttctccttc gggctgtaat tagcgcttgg 6420
   tttaatgacg gcttgtttct tttctgtggc tgcgtgaaag ccttgagggg ctccgggagg 6480
   gccctttgtg cggggggagc ggctcggggc tgtccgcggg gggacggctg ccttcggggg 6540
   ggacggggca gggcggggtt cggcttctgg cgtgtgaccg gcggctctag agcctctgct 6600
   aaccatgttc atgccttctt ctttttccta cagctcctgg gcaacgtgct ggttattgtg 6660
   ctgtctcatc attttggcaa agaattggat cggaccgaaa ttaatacgac tcactata 6718
<210> 12
   <211> 6718
   <212> DNA
   <213> artificial
<220>
   <223> pTriEX-Mup44 [D211A]
<400> 12
   ggggaattgt gagcggataa caattccccg gagttaatcc gggaccttta attcaaccca      60
   acacaatata ttatagttaa ataagaatta ttatcaaatc atttgtatat taattaaaat     120
   actatactgt aaattacatt ttatttacaa tcaaaggaga tataccatgg aacctgggca     180
   gccccgggag ccccaggagc cccgcgagcc cgggccagga gcggagaccg ctgcggcccc     240
   ggtctgggag gaagccaaga ttttctacga caacctcgcg cccaagaaga aacccaaatc     300
   gcccaagcct cagaatgcag tcaccatcgc tgtgtcctcc cgagccttgt ttcgcatgga     360
   cgaggagcag cagatctaca cggagcaggg cgtggaggag tacgtgcgct accagctgga     420
   acatgagaac gaacccttca gtcccgggcc agccttccct tttgtgaagg ctctggaggc     480
   cgtgaacagg cggctgcggg agctgtaccc tgatagtgag gacgtcttcg acatcgtcct     540
   catgactaac aaccatgctc aagtgggtgt ccgcctcatc aacagtatca accactatga     600
   cctgttcatc gagaggttct gcatgacagg tgggaacagc ccgatctgct acctcaaggc     660
   ctatcacacc aacctctact tgtcagccga tgcggaaaaa gtgcgagaag ccattgatga     720
   ggggatcgca gctgccacca tcttcagccc cagcagggat gtggttgtgt cccagagtca     780
   gctgcgcgtg gccttcgcag gggacgccgt gctcttctcg gacgagtcgg agcgcatcgt     840
   caaggcccac gggctggacc gattcttcga gcatgagaag gcccacgaga acaaacctct     900
   ggctcagggc cccttaaagg gctttctgga ggcactgggt aggttgcaga agaagttcta     960
   ctccaaaggc ctgcggctgg agtgcccaat tcgtacctac ttggtgacag cacgcagtgc    1020
   agccagttcc ggggcccggg ctctcaagac cctgcgcagc tggggcctgg agacagatga    1080
   agccttgttc cttgctggag cgcccaaggg ccctctcctt gagaagatcc gcccacacat    1140
   cttctttgat gaccagatgt tccatgtggc tggggctcag gagatgggca ctgtggccgc    1200
   ccatgtgcct tatggtgtgg cacagacacc ccggcggact gcacctgcaa agcaggcccc    1260
   atctgcacag ctcgagcacc accatcacca tcaccatcac taagtgatta acctcaggtg    1320
   caggctgcct atcagaaggt ggtggctggt gtggccaatg ccctggctca caaataccac    1380
   tgagatcgat ctttttccct ctgccaaaaa ttatggggac atcatgaagc cccttgagca 1440
   tctgacttct ggctaataaa ggaaatttat tttcattgca atagtgtgtt ggaatttttt 1500
   gtgtctctca ctcggaagga catatgggag ggcaaatcat ttaaaacatc agaatgagta 1560
   tttggtttag agtttggcaa catatgccca tatgtaacta gcataacccc ttggggcctc 1620
   taaacgggtc ttgaggggtt ttttgctgaa agcatgcgga ggaaattctc cttgaagttt 1680
   ccctggtgtt caaagtaaag gagtttgcac cagacgcacc tctgttcact ggtccggcgt 1740
   attaaaacac gatacattgt tattagtaca tttattaagc gctagattct gtgcgttgtt 1800
   gatttacaga caattgttgt acgtatttta ataattcatt aaatttataa tctttagggt 1860
   ggtatgttag agcgaaaatc aaatgatttt cagcgtcttt atatctgaat ttaaatatta 1920
   aatcctcaat agatttgtaa aataggtttc gattagtttc aaacaagggt tgtttttccg 1980
   aaccgatggc tggactatct aatggatttt cgctcaacgc cacaaaactt gccaaatctt 2040
   gtagcagcaa tctagctttg tcgatattcg tttgtgtttt gttttgtaat aaaggttcga 2100
   cgtcgttcaa aatattatgc gcttttgtat ttctttcatc actgtcgtta gtgtacaatt 2160
   gactcgacgt aaacacgtta aatagagctt ggacatattt aacatcgggc gtgttagctt 2220
   tattaggccg attatcgtcg tcgtcccaac cctcgtcgtt agaagttgct tccgaagacg 2280
   attttgccat agccacacga cgcctattaa ttgtgtcggc taacacgtcc gcgatcaaat 2340
   ttgtagttga gctttttgga attatttctg attgcgggcg tttttgggcg ggtttcaatc 2400
   taactgtgcc cgattttaat tcagacaaca cgttagaaag cgatggtgca ggcggtggta 2460
   acatttcaga cggcaaatct actaatggcg gcggtggtgg agctgatgat aaatctacca 2520
   tcggtggagg cgcaggcggg gctggcggcg gaggcggagg cggaggtggt ggcggtgatg 2580
   cagacggcgg tttaggctca aatgtctctt taggcaacac agtcggcacc tcaactattg 2640
   tactggtttc gggcgccgtt tttggtttga ccggtctgag acgagtgcga tttttttcgt 2700
   ttctaatagc ttccaacaat tgttgtctgt cgtctaaagg tgcagcgggt tgaggttccg 2760
   tcggcattgg tggagcgggc ggcaattcag acatcgatgg tggtggtggt ggtggaggcg 2820
   ctggaatgtt aggcacggga gaaggtggtg gcggcggtgc cgccggtata atttgttctg 2880
   gtttagtttg ttcgcgcacg attgtgggca ccggcgcagg cgccgctggc tgcacaacgg 2940
   aaggtcgtct gcttcgaggc agcgcttggg gtggtggcaa ttcaatatta taattggaat 3000
   acaaatcgta aaaatctgct ataagcattg taatttcgct atcgtttacc gtgccgatat 3060
   ttaacaaccg ctcaatgtaa gcaattgtat tgtaaagaga ttgtctcaag ctcggaacgc 3120
   tgcgctcggt cgttcggctg cggcgagcgg tatcagctca ctcaaaggcg gtaatacggt 3180
   tatccacaga atcaggggat aacgcaggaa agaacatgtg agcaaaaggc cagcaaaagg 3240
   ccaggaaccg taaaaaggcc gcgttgctgg cgtttttcca taggctccgc ccccctgacg 3300
   agcatcacaa aaatcgacgc tcaagtcaga ggtggcgaaa cccgacagga ctataaagat 3360
   accaggcgtt tccccctgga agctccctcg tgcgctctcc tgttccgacc ctgccgctta 3420
   ccggatacct gtccgccttt ctcccttcgg gaagcgtggc gctttctcaa tgctcacgct 3480
   gtaggtatct cagttcggtg taggtcgttc gctccaagct gggctgtgtg cacgaacccc 3540
   ccgttcagcc cgaccgctgc gccttatccg gtaactatcg tcttgagtcc aacccggtaa 3600
   gacacgactt atcgccactg gcagcagcca ctggtaacag gattagcaga gcgaggtatg 3660
   taggcggtgc tacagagttc ttgaagtggt ggcctaacta cggctacact agaaggacag 3720
   tatttggtat ctgcgctctg ctgaagccag ttaccttcgg aaaaagagtt ggtagctctt 3780
   gatccggcaa acaaaccacc gctggtagcg gtggtttttt tgtttgcaag cagcagatta 3840
   cgcgcagaaa aaaaggatct caagaagatc ctttgttacc aatgcttaat cagtgaggca 3900
   cctatctcag cgatctgtct atttcgttca tccatagttg cctgactccc cgtcgtgtag 3960
   ataactacga tacgggaggg cttaccatct ggccccagtg ctgcaatgat accgcgagac 4020
   ccacgctcac cggctccaga tttatcagca ataaaccagc cagccggaag ggccgagcgc 4080
   agaagtggtc ctgcaacttt atccgcctcc atccagtcta ttaattgttg ccgggaagct 4140
   agagtaagta gttcgccagt taatagtttg cgcaacgttg ttgccattgc tacaggcatc 4200
   gtggtgtcac gctcgtcgtt tggtatggct tcattcagct ccggttccca acgatcaagg 4260
   cgagttacat gatcccccat gttgtgcaaa aaagcggtta gctccttcgg tcctccgatc 4320
   gttgtcagaa gtaagttggc cgcagtgtta tcactcatgg ttatggcagc actgcataat 4380
   tctcttactg tcatgccatc cgtaagatgc ttttctgtga ctggtgagta ctcaaccaag 4440
   tcattctgag aatagtgtat gcggcgaccg agttgctctt gcccggcgtc aatacgggat 4500
   aataccgcgc cacatagcag aactttaaaa gtgctcatca ttggaaaacg ttcttcgggg 4560
   cgaaaactct caaggatctt accgctgttg agatccagtt cgatgtaacc cactcgtgca 4620
   cccaactgat cttcagcatc ttttactttc accagcgttt ctgggtgagc aaaaacagga 4680
   aggcaaaatg ccgcaaaaaa gggaataagg gcgacacgga aatgttgaat actcatactc 4740
   ttcctttttc aatattattg aagcatttat cagggttatt gtctcatgtc cgcgcgtttc 4800
   ctgcatcttt taatcaaatc ccaagatgtg tataaaccac caaactgcca aaaaatgaaa 4860
   actgtcgaca agctctgtcc gtttgctggc aactgcaagg gtctcaatcc tatttgtaat 4920
   tattgaataa taaaacaatt ataaatgtca aatttgtttt ttattaacga tacaaaccaa 4980
   acgcaacaag aacatttgta gtattatcta taattgaaaa cgcgtagtta taatcgctga 5040
   ggtaatattt aaaatcattt tcaaatgatt cacagttaat ttgcgacaat ataattttat 5100
   tttcacataa actagacgcc ttgtcgtctt cttcttcgta ttccttctct ttttcatttt 5160
   tctcttcata aaaattaaca tagttattat cgtatccata tatgtatcta tcgtatagag 5220
   taaatttttt gttgtcataa atatatatgt cttttttaat ggggtgtata gtaccgctgc 5280
   gcatagtttt tctgtaattt acaacagtgc tattttctgg tagttcttcg gagtgtgttg 5340
   ctttaattat taaatttata taatcaatga atttgggatc gtcggttttg tacaatatgt 5400
   tgccggcata gtacgcagct tcttctagtt caattacacc attttttagc agcaccggat 5460
   taacataact ttccaaaatg ttgtacgaac cgttaaacaa aaacagttca cctccctttt      5520
   ctatactatt gtctgcgagc agttgtttgt tgttaaaaat aacagccatt gtaatgagac      5580
   gcacaaacta atatcacaaa ctggaaatgt ctatcaatat atagttgctc tagttattaa      5640
   tagtaatcaa ttacggggtc attagttcat agcccatata tggagttccg cgttacataa      5700
   cttacggtaa atggcccgcc tggctgaccg cccaacgacc cccgcccatt gacgtcaata      5760
   atgacgtatg ttcccatagt aacgccaata gggactttcc attgacgtca atgggtggac      5820
   tatttacggt aaactgccca cttggcagta catcaagtgt atcatatgcc aagtacgccc      5880
   cctattgacg tcaatgacgg taaatggccc gcctggcatt atgcccagta catgacctta      5940
   tgggactttc ctacttggca gtacatctac gtattagtca tcgctattac catgcatggt      6000
   cgaggtgagc cccacgttct gcttcactct ccccatctcc cccccctccc cacccccaat      6060
   tttgtattta tttatttttt aattattttg tgcagcgatg ggggcggggg gggggggggg      6120
   gcgcgcgcca ggcggggcgg ggcggggcga ggggcggggc ggggcgaggc ggagaggtgc      6180
   ggcggcagcc aatcagagcg gcgcgctccg aaagtttcct tttatggcga ggcggcggcg      6240
   gcggcggccc tataaaaagc gaagcgcgcg gcgggcggga gtcgctgcga cgctgccttc      6300
   gccccgtgcc ccgctccgcc gccgcctcgc gccgcccgcc ccggctctga ctgaccgcgt      6360
   tactcccaca ggtgagcggg cgggacggcc cttctccttc gggctgtaat tagcgcttgg      6420
   tttaatgacg gcttgtttct tttctgtggc tgcgtgaaag ccttgagggg ctccgggagg      6480
   gccctttgtg cggggggagc ggctcggggc tgtccgcggg gggacggctg ccttcggggg      6540
   ggacggggca gggcggggtt cggcttctgg cgtgtgaccg gcggctctag agcctctgct      6600
   aaccatgttc atgccttctt ctttttccta cagctcctgg gcaacgtgct ggttattgtg      6660
   ctgtctcatc attttggcaa agaattggat cggaccgaaa ttaatacgac tcactata        6718
<210> 13
   <211> 6718
   <212> DNA
   <213> artificial
<220>
   <223> pTriEX-Mup44 (opt.)
<400> 13
   ggggaattgt gagcggataa caattccccg gagttaatcc gggaccttta attcaaccca 60
   acacaatata ttatagttaa ataagaatta ttatcaaatc atttgtatat taattaaaat 120
   actatactgt aaattacatt ttatttacaa tcaaaggaga tataccatgg aaccaggtca 180
   acctagagaa cctcaggaac ctagggaacc aggtccagga gctgaaacag ctgctgctcc 240
   agtgtgggag gaagcgaaaa tcttctacga caatctggca cccaagaaga aacccaagag 300
   tccgaaaccc cagaatgccg tgacaattgc cgtgtcaagc agggctctgt tccgaatgga 360
   cgaagaacag cagatctaca ccgagcaagg cgtggaagaa tacgttcgct atcagctcga 420
   acacgaaaat gaaccgttca gccctggacc cgcatttccc tttgttaaag ccctggaagc 480
   ggtcaacagg agactgaggg agctttaccc tgattctgag gacgtctttg acatcgtgct      540
   gatgacaaac aaccatgctc aggtgggagt tcgcctgatc aactccatca accactatga      600
   cctgtttata gagcgctttt gcatgaccgg tggaaactcc ccaatttgct acctcaaagc      660
   ctatcacacc aatctgtacc tttccgctga tgccgagaag gtccgagagg caatcgatga      720
   gggtattgca gcagctacga tcttcagtcc atccagagat gtcgtggtgt ctcaaagcca      780
   gctgagagtg gcgtttgatg gagatgccgt actcttcagc gacgagtcag agcgtattgt      840
   gaaggcacat gggctcgacc ggttctttga gcatgagaag gcccacgaga ataagcctct      900
   tgcccaagga cctctgaaag ggtttctgga ggcactgggc aggctgcaga agaagttcta      960
   tagcaaaggt ttgcgcttgg agtgtccaat acggacctat ctcgtcacag ctagatctgc     1020
   tgcaagcagt ggagcccgag ctctgaaaac gttgcgttca tggggccttg agactgacga     1080
   ggctctcttt ctggctggag ctcccaaagg tcctctgttg gagaagatac ggcctcacat     1140
   tttcttcgat gaccagatgt tccacgttgc aggtgctcag gagatgggca ccgtagccgc     1200
   tcatgtgcca tacggagtag cgcaaactcc caggagaact gcaccagcca agcaggcacc     1260
   ctctgcacag ctcgagcacc accatcacca tcaccatcac taagtgatta acctcaggtg     1320
   caggctgcct atcagaaggt ggtggctggt gtggccaatg ccctggctca caaataccac     1380
   tgagatcgat ctttttccct ctgccaaaaa ttatggggac atcatgaagc cccttgagca     1440
   tctgacttct ggctaataaa ggaaatttat tttcattgca atagtgtgtt ggaatttttt     1500
   gtgtctctca ctcggaagga catatgggag ggcaaatcat ttaaaacatc agaatgagta     1560
   tttggtttag agtttggcaa catatgccca tatgtaacta gcataacccc ttggggcctc     1620
   taaacgggtc ttgaggggtt ttttgctgaa agcatgcgga ggaaattctc cttgaagttt     1680
   ccctggtgtt caaagtaaag gagtttgcac cagacgcacc tctgttcact ggtccggcgt     1740
   attaaaacac gatacattgt tattagtaca tttattaagc gctagattct gtgcgttgtt     1800
   gatttacaga caattgttgt acgtatttta ataattcatt aaatttataa tctttagggt     1860
   ggtatgttag agcgaaaatc aaatgatttt cagcgtcttt atatctgaat ttaaatatta     1920
   aatcctcaat agatttgtaa aataggtttc gattagtttc aaacaagggt tgtttttccg     1980
   aaccgatggc tggactatct aatggatttt cgctcaacgc cacaaaactt gccaaatctt     2040
   gtagcagcaa tctagctttg tcgatattcg tttgtgtttt gttttgtaat aaaggttcga     2100
   cgtcgttcaa aatattatgc gcttttgtat ttctttcatc actgtcgtta gtgtacaatt     2160
   gactcgacgt aaacacgtta aatagagctt ggacatattt aacatcgggc gtgttagctt     2220
   tattaggccg attatcgtcg tcgtcccaac cctcgtcgtt agaagttgct tccgaagacg     2280
   attttgccat agccacacga cgcctattaa ttgtgtcggc taacacgtcc gcgatcaaat     2340
   ttgtagttga gctttttgga attatttctg attgcgggcg tttttgggcg ggtttcaatc     2400
   taactgtgcc cgattttaat tcagacaaca cgttagaaag cgatggtgca ggcggtggta     2460
   acatttcaga cggcaaatct actaatggcg gcggtggtgg agctgatgat aaatctacca     2520
   tcggtggagg cgcaggcggg gctggcggcg gaggcggagg cggaggtggt ggcggtgatg    2580
   cagacggcgg tttaggctca aatgtctctt taggcaacac agtcggcacc tcaactattg    2640
   tactggtttc gggcgccgtt tttggtttga ccggtctgag acgagtgcga tttttttcgt     2700
   ttctaatagc ttccaacaat tgttgtctgt cgtctaaagg tgcagcgggt tgaggttccg    2760
   tcggcattgg tggagcgggc ggcaattcag acatcgatgg tggtggtggt ggtggaggcg    2820
   ctggaatgtt aggcacggga gaaggtggtg gcggcggtgc cgccggtata atttgttctg    2880
   gtttagtttg ttcgcgcacg attgtgggca ccggcgcagg cgccgctggc tgcacaacgg    2940
   aaggtcgtct gcttcgaggc agcgcttggg gtggtggcaa ttcaatatta taattggaat    3000
   acaaatcgta aaaatctgct ataagcattg taatttcgct atcgtttacc gtgccgatat    3060
   ttaacaaccg ctcaatgtaa gcaattgtat tgtaaagaga ttgtctcaag ctcggaacgc    3120
   tgcgctcggt cgttcggctg cggcgagcgg tatcagctca ctcaaaggcg gtaatacggt    3180
   tatccacaga atcaggggat aacgcaggaa agaacatgtg agcaaaaggc cagcaaaagg    3240
   ccaggaaccg taaaaaggcc gcgttgctgg cgtttttcca taggctccgc ccccctgacg    3300
   agcatcacaa aaatcgacgc tcaagtcaga ggtggcgaaa cccgacagga ctataaagat    3360
   accaggcgtt tccccctgga agctccctcg tgcgctctcc tgttccgacc ctgccgctta    3420
   ccggatacct gtccgccttt ctcccttcgg gaagcgtggc gctttctcaa tgctcacgct    3480
   gtaggtatct cagttcggtg taggtcgttc gctccaagct gggctgtgtg cacgaacccc    3540
   ccgttcagcc cgaccgctgc gccttatccg gtaactatcg tcttgagtcc aacccggtaa    3600
   gacacgactt atcgccactg gcagcagcca ctggtaacag gattagcaga gcgaggtatg    3660
   taggcggtgc tacagagttc ttgaagtggt ggcctaacta cggctacact agaaggacag    3720
   tatttggtat ctgcgctctg ctgaagccag ttaccttcgg aaaaagagtt ggtagctctt    3780
   gatccggcaa acaaaccacc gctggtagcg gtggtttttt tgtttgcaag cagcagatta    3840
   cgcgcagaaa aaaaggatct caagaagatc ctttgttacc aatgcttaat cagtgaggca    3900
   cctatctcag cgatctgtct atttcgttca tccatagttg cctgactccc cgtcgtgtag    3960
   ataactacga tacgggaggg cttaccatct ggccccagtg ctgcaatgat accgcgagac    4020
   ccacgctcac cggctccaga tttatcagca ataaaccagc cagccggaag ggccgagcgc    4080
   agaagtggtc ctgcaacttt atccgcctcc atccagtcta ttaattgttg ccgggaagct    4140
   agagtaagta gttcgccagt taatagtttg cgcaacgttg ttgccattgc tacaggcatc    4200
   gtggtgtcac gctcgtcgtt tggtatggct tcattcagct ccggttccca acgatcaagg    4260
   cgagttacat gatcccccat gttgtgcaaa aaagcggtta gctccttcgg tcctccgatc    4320
   gttgtcagaa gtaagttggc cgcagtgtta tcactcatgg ttatggcagc actgcataat    4380
   tctcttactg tcatgccatc cgtaagatgc ttttctgtga ctggtgagta ctcaaccaag    4440
   tcattctgag aatagtgtat gcggcgaccg agttgctctt gcccggcgtc aatacgggat    4500
   aataccgcgc cacatagcag aactttaaaa gtgctcatca ttggaaaacg ttcttcgggg    4560
   cgaaaactct caaggatctt accgctgttg agatccagtt cgatgtaacc cactcgtgca 4620
   cccaactgat cttcagcatc ttttactttc accagcgttt ctgggtgagc aaaaacagga 4680
   aggcaaaatg ccgcaaaaaa gggaataagg gcgacacgga aatgttgaat actcatactc 4740
   ttcctttttc aatattattg aagcatttat cagggttatt gtctcatgtc cgcgcgtttc 4800
   ctgcatcttt taatcaaatc ccaagatgtg tataaaccac caaactgcca aaaaatgaaa 4860
   actgtcgaca agctctgtcc gtttgctggc aactgcaagg gtctcaatcc tatttgtaat 4920
   tattgaataa taaaacaatt ataaatgtca aatttgtttt ttattaacga tacaaaccaa 4980
   acgcaacaag aacatttgta gtattatcta taattgaaaa cgcgtagtta taatcgctga 5040
   ggtaatattt aaaatcattt tcaaatgatt cacagttaat ttgcgacaat ataattttat 5100
   tttcacataa actagacgcc ttgtcgtctt cttcttcgta ttccttctct tttcattttt 5160
   tctcttcata aaaattaaca tagttattat cgtatccata tatgtatcta tcgtatagag 5220
   taaatttttt gttgtcataa atatatatgt cttttttaat ggggtgtata gtaccgctgc 5280
   gcatagtttt tctgtaattt acaacagtgc tattttctgg tagttcttcg gagtgtgttg 5340
   ctttaattat taaatttata taatcaatga atttgggatc gtcggttttg tacaatatgt 5400
   tgccggcata gtacgcagct tcttctagtt caattacacc attttttagc agcaccggat 5460
   taacataact ttccaaaatg ttgtacgaac cgttaaacaa aaacagttca cctccctttt 5520
   ctatactatt gtctgcgagc agttgtttgt tgttaaaaat aacagccatt gtaatgagac 5580
   gcacaaacta atatcacaaa ctggaaatgt ctatcaatat atagttgctc tagttattaa 5640
   tagtaatcaa ttacggggtc attagttcat agcccatata tggagttccg cgttacataa 5700
   cttacggtaa atggcccgcc tggctgaccg cccaacgacc cccgcccatt gacgtcaata 5760
   atgacgtatg ttcccatagt aacgccaata gggactttcc attgacgtca atgggtggac 5820
   tatttacggt aaactgccca cttggcagta catcaagtgt atcatatgcc aagtacgccc 5880
   cctattgacg tcaatgacgg taaatggccc gcctggcatt atgcccagta catgacctta 5940
   tgggactttc ctacttggca gtacatctac gtattagtca tcgctattac catgcatggt 6000
   cgaggtgagc cccacgttct gcttcactct ccccatctcc cccccctccc cacccccaat 6060
   tttgtattta tttatttttt aattattttg tgcagcgatg ggggcggggg gggggggggg 6120
   gcgcgcgcca ggcggggcgg ggcggggcga ggggcggggc ggggcgaggc ggagaggtgc 6180
   ggcggcagcc aatcagagcg gcgcgctccg aaagtttcct tttatggcga ggcggcggcg 6240
   gcggcggccc tataaaaagc gaagcgcgcg gcgggcggga gtcgctgcga cgctgccttc 6300
   gccccgtgcc ccgctccgcc gccgcctcgc gccgcccgcc ccggctctga ctgaccgcgt 6360
   tactcccaca ggtgagcggg cgggacggcc cttctccttc gggctgtaat tagcgcttgg 6420
   tttaatgacg gcttgtttct tttctgtggc tgcgtgaaag ccttgagggg ctccgggagg 6480
   gccctttgtg cggggggagc ggctcggggc tgtccgcggg gggacggctg ccttcggggg 6540
   ggacggggca gggcggggtt cggcttctgg cgtgtgaccg gcggctctag agcctctgct 6600
   aaccatgttc atgccttctt ctttttccta cagctcctgg gcaacgtgct ggttattgtg 6660
   ctgtctcatc attttggcaa agaattggat cggaccgaaa ttaatacgac tcactata 6718
<210> 14
   <211> 6593
   <212> DNA
   <213> artificial
<220>
   <223> pTriEX-Mup44 (opt.) [D211A]
<400> 14
   actgtaaatt acattttatt tacaatcaaa ggagatatac catggaacca ggtcaaccta      60
   gagaacctca ggaacctagg gaaccaggtc caggagctga aacagctgct gctccagtgt     120
   gggaggaagc gaaaatcttc tacgacaatc tggcacccaa gaagaaaccc aagagtccga     180
   aaccccagaa tgccgtgaca attgccgtgt caagcagggc tctgttccga atggacgaag     240
   aacagcagat ctacaccgag caaggcgtgg aagaatacgt tcgctatcag ctcgaacacg     300
   aaaatgaacc gttcagccct ggacccgcat ttccctttgt taaagccctg gaagcggtca     360
   acaggagact gagggagctt taccctgatt ctgaggacgt ctttgacatc gtgctgatga     420
   caaacaacca tgctcaggtg ggagttcgcc tgatcaactc catcaaccac tatgacctgt     480
   ttatagagcg cttttgcatg accggtggaa actccccaat ttgctacctc aaagcctatc     540
   acaccaatct gtacctttcc gctgatgccg agaaggtccg agaggcaatc gatgagggta     600
   ttgcagcagc tacgatcttc agtccatcca gagatgtcgt ggtgtctcaa agccagctga     660
   gagtggcgtt tgcaggagat gccgtactct tcagcgacga gtcagagcgt attgtgaagg     720
   cacatgggct cgaccggttc tttgagcatg agaaggccca cgagaataag cctcttgccc     780
   aaggacctct gaaagggttt ctggaggcac tgggcaggct gcagaagaag ttctatagca     840
   aaggtttgcg cttggagtgt ccaatacgga cctatctcgt cacagctaga tctgctgcaa     900
   gcagtggagc ccgagctctg aaaacgttgc gttcatgggg ccttgagact gacgaggctc     960
   tctttctggc tggagctccc aaaggtcctc tgttggagaa gatacggcct cacattttct    1020
   tcgatgacca gatgttccac gttgcaggtg ctcaggagat gggcaccgta gccgctcatg    1080
   tgccatacgg agtagcgcaa actcccagga gaactgcacc agccaagcag gcaccctctg    1140
   cacagctcga gcaccaccat caccatcacc atcactaagt gattaacctc aggtgcaggc    1200
   tgcctatcag aaggtggtgg ctggtgtggc caatgccctg gctcacaaat accactgaga    1260
   tcgatctttt tccctctgcc aaaaattatg gggacatcat gaagcccctt gagcatctga    1320
   cttctggcta ataaaggaaa tttattttca ttgcaatagt gtgttggaat tttttgtgtc    1380
   tctcactcgg aaggacatat gggagggcaa atcatttaaa acatcagaat gagtatttgg    1440
   tttagagttt ggcaacatat gcccatatgt aactagcata accccttggg gcctctaaac    1500
   gggtcttgag ggtttttttg ctgaaagcat gcggaggaaa ttctccttga agtttccctg    1560
   gtgttcaaag taaaggagtt tgcaccagac gcacctctgt tcactggtcc ggcgtattaa    1620
   aacacgatac attgttatta gtacatttat taagcgctag attctgtgcg ttgttgattt 1680
   acagacaatt gttgtacgta ttttaataat tcattaaatt tataatcttt agggtggtat 1740
   gttagagcga aaatcaaatg attttcagcg tctttatatc tgaatttaaa tattaaatcc 1800
   tcaatagatt tgtaaaatag gtttcgatta gtttcaaaca agggttgttt ttccgaaccg 1860
   atggctggac tatctaatgg attttcgctc aacgccacaa aacttgccaa atcttgtagc 1920
   agcaatctag ctttgtcgat attcgtttgt gttttgtttt gtaataaagg ttcgacgtcg 1980
   ttcaaaatat tatgcgcttt tgtatttctt tcatcactgt cgttagtgta caattgactc 2040
   gacgtaaaca cgttaaatag agcttggaca tatttaacat cgggcgtgtt agctttatta 2100
   ggccgattat cgtcgtcgtc ccaaccctcg tcgttagaag ttgcttccga agacgatttt 2160
   gccatagcca cacgacgcct attaattgtg tcggctaaca cgtccgcgat caaatttgta 2220
   gttgagcttt ttggaattat ttctgattgc gggcgttttt gggcgggttt caatctaact 2280
   gtgcccgatt ttaattcaga caacacgtta gaaagcgatg gtgcaggcgg tggtaacatt 2340
   tcagacggca aatctactaa tggcggcggt ggtggagctg atgataaatc taccatcggt 2400
   ggaggcgcag gcggggctgg cggcggaggc ggaggcggag gtggtggcgg tgatgcagac 2460
   ggcggtttag gctcaaatgt ctctttaggc aacacagtcg gcacctcaac tattgtactg 2520
   gtttcgggcg ccgtttttgg tttgaccggt ctgagacgag tgcgattttt ttcgtttcta 2580
   atagcttcca acaattgttg tctgtcgtct aaaggtgcag cgggttgagg ttccgtcggc 2640
   attggtggag cgggcggcaa ttcagacatc gatggtggtg gtggtggtgg aggcgctgga 2700
   atgttaggca cgggagaagg tggtggcggc ggtgccgccg gtataatttg ttctggttta 2760
   gtttgttcgc gcacgattgt gggcaccggc gcaggcgccg ctggctgcac aacggaaggt 2820
   cgtctgcttc gaggcagcgc ttggggtggt ggcaattcaa tattataatt ggaatacaaa 2880
   tcgtaaaaat ctgctataag cattgtaatt tcgctatcgt ttaccgtgcc gatatttaac 2940
   aaccgctcaa tgtaagcaat tgtattgtaa agagattgtc tcaagctcgg aacgctgcgc 3000
   tcggtcgttc ggctgcggcg agcggtatca gctcactcaa aggcggtaat acggttatcc 3060
   acagaatcag gggataacgc aggaaagaac atgtgagcaa aaggccagca aaaggccagg 3120
   aaccgtaaaa aggccgcgtt gctggcgttt ttccataggc tccgcccccc tgacgagcat 3180
   cacaaaaatc gacgctcaag tcagaggtgg cgaaacccga caggactata aagataccag 3240
   gcgtttcccc ctggaagctc cctcgtgcgc tctcctgttc cgaccctgcc gcttaccgga 3300
   tacctgtccg cctttctccc ttcgggaagc gtggcgcttt ctcaatgctc acgctgtagg 3360
   tatctcagtt cggtgtaggt cgttcgctcc aagctgggct gtgtgcacga accccccgtt 3420
   cagcccgacc gctgcgcctt atccggtaac tatcgtcttg agtccaaccc ggtaagacac 3480
   gacttatcgc cactggcagc agccactggt aacaggatta gcagagcgag gtatgtaggc 3540
   ggtgctacag agttcttgaa gtggtggcct aactacggct acactagaag gacagtattt 3600
   ggtatctgcg ctctgctgaa gccagttacc ttcggaaaaa gagttggtag ctcttgatcc 3660
   ggcaaacaaa ccaccgctgg tagcggtggt ttttttgttt gcaagcagca gattacgcgc 3720
   agaaaaaaag gatctcaaga agatcctttg ttaccaatgc ttaatcagtg aggcacctat 3780
   ctcagcgatc tgtctatttc gttcatccat agttgcctga ctccccgtcg tgtagataac 3840
   tacgatacgg gagggcttac catctggccc cagtgctgca atgataccgc gagacccacg 3900
   ctcaccggct ccagatttat cagcaataaa ccagccagcc ggaagggccg agcgcagaag 3960
   tggtcctgca actttatccg cctccatcca gtctattaat tgttgccggg aagctagagt 4020
   aagtagttcg ccagttaata gtttgcgcaa cgttgttgcc attgctacag gcatcgtggt 4080
   gtcacgctcg tcgtttggta tggcttcatt cagctccggt tcccaacgat caaggcgagt 4140
   tacatgatcc cccatgttgt gcaaaaaagc ggttagctcc ttcggtcctc cgatcgttgt 4200
   cagaagtaag ttggccgcag tgttatcact catggttatg gcagcactgc ataattctct 4260
   tactgtcatg ccatccgtaa gatgcttttc tgtgactggt gagtactcaa ccaagtcatt 4320
   ctgagaatag tgtatgcggc gaccgagttg ctcttgcccg gcgtcaatac gggataatac 4380
   cgcgccacat agcagaactt taaaagtgct catcattgga aaacgttctt cggggcgaaa 4440
   actctcaagg atcttaccgc tgttgagatc cagttcgatg taacccactc gtgcacccaa 4500
   ctgatcttca gcatctttta ctttcaccag cgtttctggg tgagcaaaaa caggaaggca 4560
   aaatgccgca aaaaagggaa taagggcgac acggaaatgt tgaatactca tactcttcct 4620
   ttttcaatat tattgaagca tttatcaggg ttattgtctc atgtccgcgc gtttcctgca 4680
   tcttttaatc aaatcccaag atgtgtataa accaccaaac tgccaaaaaa tgaaaactgt 4740
   cgacaagctc tgtccgtttg ctggcaactg caagggtctc aatcctattt gtaattattg 4800
   aataataaaa caattataaa tgtcaaattt gttttttatt aacgatacaa accaaacgca 4860
   acaagaacat ttgtagtatt atctataatt gaaaacgcgt agttataatc gctgaggtaa 4920
   tatttaaaat cattttcaaa tgattcacag ttaatttgcg acaatataat tttattttca 4980
   cataaactag acgccttgtc gtcttcttct tcgtattcct tctctttttc atttttctct 5040
   tcataaaaat taacatagtt attatcgtat ccatatatgt atctatcgta tagagtaaat 5100
   tttttgttgt cataaatata tatgtctttt ttaatggggt gtatagtacc gctgcgcata 5160
   gtttttctgt aatttacaac agtgctattt tctggtagtt cttcggagtg tgttgcttta 5220
   attattaaat ttatataatc aatgaatttg ggatcgtcgg ttttgtacaa tatgttgccg 5280
   gcatagtacg cagcttcttc tagttcaatt acaccatttt ttagcagcac cggattaaca 5340
   taactttcca aaatgttgta cgaaccgtta aacaaaaaca gttcacctcc cttttctata 5400
   ctattgtctg cgagcagttg tttgttgtta aaaataacag ccattgtaat gagacgcaca 5460
   aactaatatc acaaactgga aatgtctatc aatatatagt tgctctagtt attaatagta 5520
   atcaattacg gggtcattag ttcatagccc atatatggag ttccgcgtta cataacttac 5580
   ggtaaatggc ccgcctggct gaccgcccaa cgacccccgc ccattgacgt caataatgac 5640
   gtatgttccc atagtaacgc caatagggac tttccattga cgtcaatggg tggactattt 5700
   acggtaaact gcccacttgg cagtacatca agtgtatcat atgccaagta cgccccctat 5760
   tgacgtcaat gacggtaaat ggcccgcctg gcattatgcc cagtacatga ccttatggga 5820
   ctttcctact tggcagtaca tctacgtatt agtcatcgct attaccatgc atggtcgagg 5880
   tgagccccac gttctgcttc actctcccca tctccccccc ctccccaccc ccaattttgt 5940
   atttatttat tttttaatta ttttgtgcag cgatgggggc gggggggggg ggggggcgcg 6000
   cgccaggcgg ggcggggcgg ggcgaggggc ggggcggggc gaggcggaga ggtgcggcgg 6060
   cagccaatca gagcggcgcg ctccgaaagt ttccttttat ggcgaggcgg cggcggcggc 6120
   ggccctataa aaagcgaagc gcgcggcggg cgggagtcgc tgcgacgctg ccttcgcccc 6180
   gtgccccgct ccgccgccgc ctcgcgccgc ccgccccggc tctgactgac cgcgttactc 6240
   ccacaggtga gcgggcggga cggcccttct ccttcgggct gtaattagcg cttggtttaa 6300
   tgacggcttg tttcttttct gtggctgcgt gaaagccttg aggggctccg ggagggccct 6360
   ttgtgcgggg ggagcggctc ggggctgtcc gcggggggac ggctgccttc gggggggacg 6420
   gggcagggcg gggttcggct tctggcgtgt gaccggcggc tctagagcct ctgctaacca 6480
   tgttcatgcc ttcttctttt tcctacagct cctgggcaac gtgctggtta ttgtgctgtc 6540
   tcatcatttt ggcaaagaat tggatcggac cgaaattaat acgactcact ata 6593
<210> 15
   <211> 70
   <212> DNA
   <213> artificial
<220>
   <223> 5' region of the nucleotide sequence encoding CN-1A
<400> 15
   cctgggcagc cccgggagcc ccaggagccc cgcgagcccg ggccaggagc ggagaccgct 60
   gcggccccgg 70
<210> 16
   <211> 367
   <212> PRT
   <213> artificial
<220>
   <223> immunogenic wild type sequence of cN-1A having a lower degree of enzymatic activity without His tag
<400> 16
   Met Glu Pro Gly Gln Pro Arg Glu Pro Gln Glu Pro Arg Glu Pro Gly
   1               5                   10                  15
   Pro Gly Ala Glu Thr Ala Ala Ala Pro Val Trp Glu Glu Ala Lys Ile
               20                  25                  30
   Phe Tyr Asp Asn Leu Ala Pro Lys Lys Lys Pro Lys Ser Pro Lys Pro
           35                  40                  45
   Gln Asn Ala Val Thr Ile Ala Val Ser Ser Arg Ala Leu Phe Arg Met
   50 55 60
   Asp Glu Glu Gln Gln Ile Tyr Thr Glu Gln Gly Val Glu Glu Tyr Val
   65 70 75 80
   Arg Tyr Gln Leu Glu His Glu Asn Glu Pro Phe Ser Pro Gly Pro Ala
   85 90 95
   Phe Pro Phe Val Lys Ala Leu Glu Ala Val Asn Arg Arg Leu Arg Glu
   100 105 110
   Leu Tyr Pro Asp Ser Glu Asp Val Phe Asp Ile Val Leu Met Thr Asn
   115 120 125
   Asn His Ala Gln Val Gly Val Arg Leu Ile Asn Ser Ile Asn His Tyr
   130 135 140
   Asp Leu Phe Ile Glu Arg Phe Cys Met Thr Gly Gly Asn Ser Pro Ile
   145 150 155 160
   Cys Tyr Leu Lys Ala Tyr His Thr Asn Leu Tyr Leu Ser Ala Asp Ala
   165 170 175
   Glu Lys Val Arg Glu Ala Ile Asp Glu Gly Ile Ala Ala Ala Thr Ile
   180 185 190
   Phe Ser Pro Ser Arg Asp Val Val Val Ser Gln Ser Gln Leu Arg Val
   195 200 205
   Ala Phe Ala Gly Asp Ala Val Leu Phe Ser Asp Glu Ser Glu Arg Ile
   210 215 220
   Val Lys Ala His Gly Leu Asp Arg Phe Phe Glu His Glu Lys Ala His
   225 230 235 240
   Glu Asn Lys Pro Leu Ala Gln Pro Leu Lys Gly Phe Leu Glu Ala Leu
   245 250 255
   Gly Arg Leu Gln Lys Lys Phe Tyr Ser Lys Gly Leu Arg Leu Glu Cys
   260 265 270
   Pro Ile Arg Thr Tyr Leu Val Thr Ala Arg Ser Ala Ala Ser Ser Gly
   275 280 285
   Ala Arg Ala Leu Lys Thr Leu Arg Ser Trp Gly Leu Glu Thr Asp Glu
   290 295 300
   Ala Leu Phe Leu Ala Gly Ala Pro Lys Gly Pro Leu Leu Glu Lys Ile
   305 310 315 320
   Arg Pro His Ile Phe Phe Asp Asp Gln Met Phe His Val Ala Gly Ala
                   325                 330                 335
   Gln Glu Met Gly Thr Val Ala Ala His Val Pro Tyr Gly Val Ala Gln
               340                 345                 350
   Thr Pro Arg Arg Thr Ala Pro Ala Lys Gln Ala Pro Ser Ala Gln
           355                 360                 365

## Description

The present invention relates to a polypeptide comprising a stretch of at least 150 successive amino acids from SEQ ID NO1 or a variant thereof, which polypeptide has the ability to bind specifically to an autoantibody to SEQ ID NO1, wherein the polypeptide or the variant comprises a mutation at Aspartate 211 and the Aspartate 211 is replaced by an amino acid residue other than glutamate, wherein the variant is at least 80% identical to the sequence according to SEQ ID NO1; a method comprising the step a) producing the polypeptide according to the present invention; a kit comprising the polypeptide, the cell or the carrier according to the present invention; a use of the polypeptide, the cell or the carrier according to the present invention for diagnosing a disease in vitro and a pharmaceutical composition comprising the polypeptide, the cell and preferably a pharmaceutically acceptable carrier.

Idiopathic inflammatory myopathies (IIMs) comprise a diverse group of inflammatory muscle diseases with an insidious onset characterised by chronic muscle weakness, inflammation of skeletal muscle, electromyographic abnormalities and increases in muscle enzymes. The three forms of IIM, polymyositis (PM) and dermatomyositis (DM) and inclusion body myositis IBM, are well recognised as autoimmune diseases. They exhibit an array of autoantibodies directed against ubiquitous intracellular antigens that can be either myositis-specific or myositis-associated.

IBM is supposedly the most common acquired muscle disease in adults aged >50 years, characterized clinically by an insidious onset of muscle weakness and muscle atrophy that slowly leads to severe disability. Unlike PM and DM, IBM is largely refractory to treatment with immunosuppressive, immunomodulatory or other therapies.

In the case of IBM, the only recognized target antigen of these autoantibodies is cytosolic 5'-nucleotidase 1A (cN-1A), and the association of these autoantibodies with the disease has led to the establishment of an assay for the diagnosis of IBM and for differentiating between the various types of inflammatory muscle diseases based on the detection of its presence in samples from patients suspected of suffering from such a disease (WO2013/006059).

ELISA is the method of choice for carrying out the assay. However, a large-scale production of adequate diagnostic devices requires huge quantities of reasonably pure cN-1A with the ability of binding to the autoantibodies. The inventors of the assay have isolated the polypeptide from human muscle lysate.

Microarrays were then set up using a set of overlapping synthetic peptides covering the complete amino acid sequence of cN-1A (WO2013/006059), and the chemical synthesis of peptides remains the most efficient method for the large-scale production of antigen for ELISA plates (Herbert et al. (2014), Disease specificity of autoantibodies to cytosolic 5'-nucleotidase 1A in sporadic inclusion body myositis versus known autoimmune diseases, Ann. Rheum. Dis., doi: 10.1136/annrhemdis-2014-206691).

Other groups also used peptide libraries for generating sufficiently active antigen (Larman et al. (2013) Ann Neurol., doi: 10.1002/ana.23840).

WO2013/006059 A1 discloses a method of diagnosing idiopathic inflammatory myopathy (IIM) and inclusion body myositis (IBM) by detecting autoantibodies against 5'-nucleotidase (cN1A).

WO2015/023626 A1 discloses an assay method with an improved sensitivity for the diagnosis of IBM, wherein at least two autoantibodies against CN1A or CN1B are detected.

Needham et al. (Needham, M. et al. (2015), Clinical Neurophysiology, vol. 127, no. 3, pages 1764-1773) summarize the knowledge about sporadic inclusion body myositis.

Larman et al. (Larman, HB. et al. (2013), Annals of Neurology, vol. 73, no. 3, pages 408-418) disclose that the moderate reactivity of anti-cN1A autoantibodies was 70% sensitive and 92% specific and the high reactivity was 34% sensitive and 98% specific for diagnosis of IBM.

Van Doreen et al. (van Doreen, SHJ. et al. (2011), Autoimmun Highlights, vol. 2, no. 1, pages 5-20) disclose myositis-specific autoantibodies, wherein antibodies to cN1A are not mentioned.

Autoantibodies against cN-1A can be found in the blood of approximately 37 % of all IBM patients using the peptide-based assay. In other words, almost two thirds of the patients cannot be diagnosed this way in a reliable manner.

Therefore, the object of the present invention is to provide an improved method for the diagnosis of idiopathic inflammatory myopathies, preferably IBM, based on the detection of autoantibodies to cN-1A, more specifically a method having a higher degree of diagnostic reliability, preferably in terms of sensitivity and/or specificity.

Another object of the present invention is to provide a method for the efficient production of an antigenic cN-1A, preferably a full-length or near full-length version of the polypeptide.

The problem underlying the present invention is solved by the subject-matter of the attached independent and dependent claims.

In a first aspect, the problem is solved by a polypeptide comprising a stretch of at least 150, preferably 200, more preferably 250, more preferably 300, most preferably 350 successive amino acids from SEQ ID NO1 or a variant thereof, which polypeptide has the ability to bind specifically to an autoantibody to SEQ ID NO1 and is preferably an immobilized polypeptide, wherein the polypeptide or the variant comprises a mutation at Aspartate 211 and the Aspartate 211 is replaced by an amino acid residue other than qlutamate, wherein the variant is at least 80% identical to the sequence according to SEQ ID NO1.

In a preferred embodiment, said polypeptide comprises at least one sequence from the group comprising SEQ ID NO2, SEQ ID NO3 and SEQ ID NO4 or a variant thereof reactive to autoantibodies to SEQ ID NO1, preferably all of them.

In a preferred embodiment, said polypeptide has a lower degree of enzymatic activity than the wild type enzyme represented by SEQ ID NO1.

The number of any amino acid residue throughout this application is in line with the numbers used in SEQ ID NO1, equivalent to protein data base entry AAI03880, as online available from the NCBI protein sequence data base on the priority date.

In a preferred embodiment, the polypeptide is an isolated and/or recombinant polypeptide, preferably optimized in terms of codon usage.

In a second aspect, the problem is solved by a nucleic acid encoding the polypeptide, a vector comprising said nucleic acid or a cell comprising said nucleic acid or said vector.

In a preferred embodiment, the cell is a prokaryotic cell, preferably Gram negative bacterial cell, more preferably *E. coli,* or a eukaryotic cell, preferably a mammalian cell, most preferably a HEK293T cell.

In a 3^{rd} aspect, the problem is solved by a method comprising the step a) producing the polypeptide according to the present invention, preferably by cultivating the cell according to the present invention under conditions compatible with the expression of the polypeptide according to the present invention.

In a preferred embodiment, the method further comprises step b) recovering, preferably purifying the polypeptide or the cell according to the present invention.

In a preferred embodiment, the method further comprises step c) immobilizing the polypeptide according to the present invention or the cell according to the present invention on the surface of a diagnostically or therapeutically useful carrier.

In a preferred embodiment, the method further comprises steps d) contacting the diagnostically or therapeutically useful carrier with a liquid sample, preferably from a subject suspected of suffering from a disease, and e) capturing an autoantibody to SEQ ID NO1 or a complex comprising said autoantibody and the polypeptide or the cell according to the present invention from the sample, optionally followed by detecting said autoantibody.

In a 4^{th} aspect, the problem is solved by a diagnostically or therapeutically useful carrier comprising the polypeptide according to the present invention or the cell according to the present invention, which carrier is preferably selected from the group comprising a glass plate or slide, biochip, microtiter plate, bead, preferably magnetic bead, apharesis device, chromatography column, membrane and blot, preferably line blot, Western blot or dot blot.

In a 5^{th} aspect, the problem is solved by a kit comprising the polypeptide according to the present invention, the cell according to the present invention or the carrier according to the present invention.

In a 6^{th} aspect, the problem is solved by a use of the polypeptide according to the present invention, the cell according to the present invention or the carrier according to the present invention for diagnosing of a disease in vitro
or
by the polypeptide according to the present invention, the cell according to the present invention or the carrier according to the present invention for use in a method for the treatment of a disease or for use in an in vivo method of diagnosis.

In a 7^{th} aspect, the problem is solved by a pharmaceutical composition comprising the polypeptide according to the present invention, the cell according to the present invention and preferably a pharmaceutically acceptable carrier.

The present invention is based on the inventors' surprising finding that diagnostic assays based on antigenic polypeptides comprising full-length or near full-length cN-1A are diagnostically more reliable than those based on peptides derived from cN-1A.

The present invention is also based on the inventors' surprising finding that a variant of cN-1A having a lower degree of enzymatic activity compared to the respective wild type enzyme can be made that binds to an autoantibody to human cN-1A, but is expressed more efficiently than the wild type enzyme.

Without wishing to be bound to any theory, the inventors hypothesize that autoantibodies to human cN-1A are present in the blood of patients suffering from IBM that bind to conformational epitopes associated with the full-length natively folded polypeptides rather than peptides derived from the same.

The present invention relates to a polypeptide comprising a stretch of at least 100, more preferably 125, 150, 175, 200, 225, 250, 275, 300, 325, 350 or 360 successive amino acids from SEQ ID NO1 or a variant thereof, which polypeptide is reactive to autoantibodies to SEQ ID NO1, wherein the polypeptide or the variant comprises a mutation at Aspartate 211 and the Aspartate 211 is replaced by an amino acid residue other than glutamate, wherein the variant is at least 80% identical to the sequence according to SEQ ID NO1 and is preferably an immobilized polypeptide.

The teachings of the present invention may not only be carried out using polypeptides, in particular a polypeptide comprising SEQ ID NO1, having the exact sequences referred to in this application explicitly, but also using variants of such polypeptides.

In a preferred embodiment, the term "variant", as used herein, may refer to at least one fragment of the full-length sequence referred to, more specifically one or more amino acids which are, relative to the full-length sequence, truncated at one or both termini by one or more amino acids. Such a fragment comprises or encodes for a peptide having at least 150 or 200 successive amino acids of the original sequence or a variant thereof. The total length of the variant may be more amino acids. Variants may include full-length sequences or fragments that are at least 80, 85, 90, 92, 94, 95, 96, 97, 98 or 99 % identical to the reference amino acid sequence referred to or the corresponding fragment of said reference amino acid sequences.

In a preferred embodiment, the polypeptide and variants thereof may, in addition, comprise chemical modifications, for example isotopic labels or covalent modifications such as glycosylation, phosphorylation, acetylation, decarboxylation, citrullination, methylation, hydroxylation and the like. The person skilled in the art is familiar with methods to modify polypeptides.

The variant of the polypeptide has biological activity. In a preferred embodiment, such biological activity is the ability to bind specifically to an autoantibody to SEQ ID NO1 found in patients suffering from IBM.

The inventive polypeptide, when used to carry out the teachings of the present invention, may be provided in any form and at any degree of purification, from liquid samples, tissues or cells comprising said polypeptide in an endogenous form, more preferably cells overexpressing the polypeptide, crude or enriched lysates of such cells, to purified and/or isolated polypeptide which is optionally essentially pure. In a preferred embodiment, the polypeptide is a native polypeptide, wherein the term "native polypeptide", as used herein, refers to a folded polypeptide, more preferably to a folded polypeptide purified from tissues or cells, more preferably from mammalian cells or tissues, optionally from non-recombinant tissues or cells. In another preferred embodiment, the polypeptide is a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", "Purifying Challenging Proteins" (2009/2010), published by GE Healthcare Life Sciences, and in Burgess, R. R., Deutscher, M. P. (2009), Guide to Protein Purification). In a preferred embodiment, a polypeptide is pure if at least 60, 70, 80, 90, 95 or 99 percent of the polypeptide in the respective sample consist of said polypeptide as judged by SDS polyacrylamide gel electrophoresis followed by Coomassie blue staining and visual inspection.

The polypeptide used to carry out the inventive teachings, including any variants, is preferably designed such that it comprises epitopes recognized by and/or binds specifically to autoantibodies binding to SEQ ID NO1. Such polypeptide comprises a stretch of 150 consecutive amino acids from SEQ ID NO1, preferably one or more selected from the group comprising SEQ ID NO2, SEQ ID NO3 and SEQ ID NO4 and a variant thereof. In a preferred embodiment, the polypeptide comprises SEQ ID NO2 and SEQ ID NO3 or a variant thereof. In another preferred embodiment, the polypeptide comprises SEQ ID NO3 and SEQ ID NO4 or a variant thereof. In another preferred embodiment, the polypeptide comprises SEQ ID NO2 and SEQ ID NO4 or a variant thereof.

The person skilled in the art is familiar with guidelines used to design peptides having sufficient immunogenicity, for example those described in Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173. Briefly, it is desirable that the peptide meets as many as possible of the following requirements: (a) it has a high degree of hydrophilicity, (b) it comprises one or more residues selected from the group comprising aspartate, proline, tyrosine and phenylalanine, (c) is has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71.

In another preferred embodiment, the variant has such biological activity, but has a lower degree of enzymatic activity than the wild type enzyme represented by SEQ ID NO1. In a more preferred embodiment, the enzymatic activity is at least 5, 10, 20, 25, 50, 75, 80, 90, 95, 98, 99 or 99.1 % lower. SEQ ID NO5 represents an example of such a variant. The activity may be measured using the colorimetric assay described by Hunsucker et al. (2000), Human Cytosolic 5'-Nucleotidase I - CHARACTERIZATION AND ROLE IN NUCLEOSIDE ANALOG RESISTANCE, The Journal of Biological Chemistry 276, 10498-10504.

The person skilled in the art is familiar with routine methods that may be used to generate a variant that has the ability to bind specifically to an autoantibody to SEQ ID NO1, but has a lower degree of enzymatic activity. Preferably, an amino acid side chain from the reactive center of the enzyme is mutated, followed by an immunoassay to confirm that the reactivity towards the autoantibody is not inhibited through the introduction of the mutation. In a preferred embodiment, the variant of SEQ ID NO1 according to this embodiment is represented by SEQ ID NO5 or a variant thereof.

The inventive polypeptide, when used according to the present invention, may be provided in any kind of conformation. For example, the polypeptide may be an essentially unfolded, a partially or a fully folded polypeptide. In a preferred embodiment, the polypeptide is folded in the sense that the epitopes essential for the binding to the inventive autoantibody, or the protein or variant thereof in its entirety, adopt the fold adopted by the native protein in its natural environment. The person skilled in the art is familiar with methods suitable to determine whether or not a polypeptide is folded and if it is, which structure it has, for example limited proteolysis, NMR spectroscopy, CD spectroscopy or X-ray crystallography (see for example Banaszak L. J. (2008), Foundations of Structural Biology, Academics Press, or Teng Q. (2013), Structural Biology: Practical Applications, Springer), preferably CD spectroscopy is used.

The inventive polypeptide may be a fusion protein which comprises amino acid sequences other than those taken from SEQ ID NO1, in particular a C-terminal or N-terminal tag, which is, in a preferred embodiment, as used herein, an additional sequence motif or polypeptide having a function that has some biological or physical function and may, for example, be used to purify, immobilize, precipitate or identify the inventive polypeptide. In a more preferred embodiment, the tag is a sequence or domain capable of binding specifically to a ligand, for example a tag selected from the group comprising His tag, thioredoxin, maltose binding protein, glutathione-S-transferase, a fluorescence tag, for example from the group comprising green fluorescent protein.

The inventive polypeptide may be an immobilized polypeptide. In a preferred embodiment, the term "immobilized", as used herein, refers to a molecule bound to a solid carrier insoluble in an aqueous solution, more preferably via a covalent bond, electrostatic interactions, encapsulation or entrapment, for example by denaturing a globular polypeptide in a gel, or via hydrophobic interactions, most preferably via one or more covalent bonds. Such carrier is preferably an artificial carrier, which is not predominantly biological material such as a tissue section. Various suitable carriers, for example paper, polystyrene, metal, silicon or glass surfaces, microfluidic channels, membranes, beads such as magnetic beads, column chromatography media, biochips, polyacrylamide gels and the like have been described in the state of the art. An immobilized polypeptide may be immobilized in a reversible or irreversible manner. The polypeptide may be indirectly immobilized, for example by immobilizing an antibody or other entity having affinity to the polypeptide, followed by formation of a complex to the effect that the molecule-antibody complex is immobilized. Various ways to immobilize molecules are described in the literature, for example in Kim, D., Herr, and A. E. (2013), Protein immobilizsation techniques for microfluidic assays, Biomicrofluidics 7(4), 041501.

The sample to be analyzed has been obtained from a subject suspected of suffering or actually suffering from a disease. The subject may be an animal that produces antibodies, preferably a mammal, more preferably a human. It is essential that the sample comprises autoantibodies. Typically, the sample of a bodily fluid comprises a representative set of the entirety of the subject's immunoglobulins. However, the sample, once provided, may be subjected to further processing which may include fractionation, centrifugation, enriching or isolating the entirety of immunoglobulins or any immunoglobulin class of the subject, which may affect the relative distribution of immunoglobulins of the various classes. In a preferred embodiment, the sample is selected from the group comprising blood sample, preferably serum sample, lymph sample and CSF sample. In a preferred embodiment, the antibody to cN-1a to be detected is an IgG class antibody.

The products, methods and uses described throughout this application may be used for the diagnosis of a disease. In a preferred embodiment, the disease is an autoimmune disease characterized by the presence of autoantibodies to SEQ ID NO1. In another preferred embodiment, the disease is an inflammatory muscle disease, preferably characterised by chronic muscle weakness, inflammation of skeletal muscle, electromyographic abnormalities and increases in muscle enzymes. In another preferred embodiment, the disease is selected from the group comprising PM, DM and IBM, preferably IBM.

In a preferred embodiment, the term "diagnosis", as used herein, refers to any kind of procedure aiming to obtain information instrumental in the assessment of whether a patient suffers or is likely or more likely than the average or a comparative subject, the latter preferably having similar symptoms, to suffer from a disease in the past, at the time of the diagnosis or in the future, to find out how the disease is progressing or is likely to progress in the future or to evaluate the responsiveness of a patient with regard to a certain treatment, for example the administration of immunosuppressive drugs. In other words, the term "diagnosis" comprises not only diagnosing, but also prognosticating and/or monitoring the course of a disease.

In many cases, the mere detection, in other words determining whether or not detectable levels of the autoantibody are present in the sample, is sufficient for the diagnosis. In a preferred embodiment, the relative concentration of the antibody in the serum, compared to the level that may be found in the average healthy subject, may be determined. In a preferred embodiment, the method may involve determining whether the concentration is at least 0.1, preferably 0.2, 0.5, 1, 2, 5, 10, 20, 25, 50, 100, 200, 500, 1000, 10000 or 100000 times higher than the concentration found in the average healthy subject. If the autoantibody can be detected or is present at an elevated concentration, this indicates an increased likelihood that the patient suffers, suffered or will suffer from the disease.

The person skilled in the art will appreciate that a clinician does usually not conclude whether or not the patient suffers or is likely to suffer from a disease, condition or disorder solely on the basis of a single diagnostic parameter, but needs to take into account other aspects, for example the presence of other autoantibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred embodiment, the meaning of any symptoms or diseases referred to throughout this application is in line with the person skilled in the art's understanding as of the priority date of the present invention as evidenced by text books and scientific publications.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", i. e. a systematic diagnostic procedure considering the likelihood of a range of possible conditions on the basis of a range of diagnostic parameters.

The term "diagnosis" may also refer to a method or agent used to distinguish between two or more diseases associated with similar or identical symptoms, preferably selected from the group comprising PM, DM and IBM.

The present invention relates to a complex comprising an antibody, preferably autoantibody, binding to the inventive polypeptide. Such a complex may be used or detected as part of a method for diagnosing a disease. A liquid sample comprising antibodies from a subject may be used to practice the method.

The method according to the present invention contemplates the step a) producing the polypeptide according to the present invention, preferably by cultivating the cell according to the present invention under conditions compatible with the expression of the polypeptide. This may involve transfecting the cell with the vector according to the present invention, wherein a nucleic acid encoding the polypeptide may be placed under the control of an inducible promotor, followed by induction of the promotor.

The method may then comprise step b) recovering, preferably purifying the polypeptide such that pure polypeptide is obtain, for example by producing, as part of step a), polypeptide fused to a purification tag such as a His tag, followed by affinity chromatography.

In a preferred embodiment, the method then comprises step c) immobilizing the polypeptide according to the present invention or cell according to the present invention, on a diagnostically or therapeutically useful carrier. In another preferred embodiment, non-immobilized polypeptide is used in step d).

The method may then comprise step d) contacting said carrier under conditions that are compatible with the formation of the complex comprising said polypeptide and an antibody, preferably an autoantibody, binding to the inventive polypeptide. The liquid sample, then depleted of antibodies binding to the inventive polypeptide may be removed subsequently, optionally followed by one or more washing steps. Finally, in step a) an autoantibody to SEQ ID NO1 or a complex comprising the autoantibody and the polypeptide according to the present invention may be captured. In case non-immobilized polypeptide was used, the complex, which is then also non-immobilized, may be captured using a second polypeptide according to the present invention or an antibody also binding to the polypeptide in complex with the autoantibody or to the autoantibody, which second polypeptide or antibody is immobilized, as capture reagent.

In a preferred embodiment, the term "conditions compatible with the formation of the complex" are conditions that allow for the specific antigen-antibody interactions to build up the complex comprising the polypeptide and the antibody. In a preferred embodiment, such conditions may comprise incubating the polypeptide in sample diluted 1:100 in PBS buffer for 30 minutes at 25 °C. In a preferred embodiment, the term "autoantibody", as used herein, refers to an antibody binding specifically to an endogenous molecule of the animal, preferably mammal, which produces said autoantibody, wherein the level of such antibody is more preferably elevated compared to the average of any other antibodies binding specifically to such an endogenous molecule. In a most preferred embodiment, the autoantibody is an autoantibody binding to SEQ ID NO1.

In a preferred embodiment, the term "antibody", as used herein, refers to any immunoglobulin-based binding moieties, more preferably one comprising at least one immunoglobulin heavy chain and one immunoglobulin light chain, including, but not limited to monoclonal and polyclonal antibodies as well as variants of an antibody, in particular fragments, which binding moieties are capable of binding to the respective antigen, more preferably binding specifically to it. In a preferred embodiment, the antibody binds specifically to its target. The term "binding specifically", as used herein, means that the binding is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7. The antibody may be part of an autoantibody preparation which is heterogeneous or may be a homogenous autoantibody, wherein a heterogeneous preparation comprises a plurality of different autoantibody species as obtainable by preparation from the sera of human donors, for example by affinity chromatography using the immobilized antigen to purify any autoantibody capable of binding to said antigen. The antibody may be glycosylated or non-glycosylated.

Finally, the method may comprise the step detecting the autoantibody or the complex comprising the autoantibody and the polypeptide according to the present invention. In a preferred embodiment, the detection is carried out using a method selected from the group comprising immunodiffusion techniques, immunoelectrophoretic techniques, light scattering immunoassays, equilibrium dialysis, agglutination techniques, labeled immunoassays such as those from the group comprising radiolabeled immunoassay, enzyme immunoassays, preferably ELISA, chemiluminscence immunoassays, and immunofluorescence techniques, spectroscopy preferably using a technique from the group comprising fluorescence, CD, NMR or EPR spectroscopy, analytical ultracentrifugation, mass spectrometry, gel chromatography or detection via a biosensor. In a preferred embodiment, a microplate, ELISA membrane, dot blot, or line blot is used to carry out the diagnostic method according to the invention.

If the detection is carried out using a solid-phase immunoassay such as a labeled solid-phase immunoassay, the immunoassay may be selected from the group comprising a sandwich immunoassay, a bridge immunoassay and a competitive immunoassay. In a preferred embodiment, a sandwich immunoassay involves immobilizing the polypeptide according to the present invention using a first antibody, preferably a monoclonal antibody, followed by contacting of the immobilized polypeptide and the sample comprising autoantibodies, followed by detection of the complex comprising the first antibody, the polypeptide and the autoantibody. In a preferred embodiment, a bridge immunoassay involves contacting a first polypeptide according to the present invention and a second polypeptide according to the present invention, the latter comprising a label, and the sample, followed by formation of a complex comprising the first polypeptide, the second polypeptide and the autoantibody from the sample, which may then be detected, optionally after immobilization of the complex. In a preferred embodiment, a competitive immunoassay involves immobilizing an antibody, contacting the antibody with a first polypeptide according to the present invention followed by contacting the antibody with a second polypeptide according to the present invention capable of displacing the first polypeptide, wherein either the first or the second polypeptide comprises a label that may be used to distinguish the first and the second polypeptide whilst bound to the antibody.

The label may be selected from the group comprising an enzymatically active label, a radioactive label, a spin label, an NMR active label, a chemiluminescent label, a microparticle label and a fluorescence label.

If an antibody such as a monoclonal or autoantibody to the polypeptide according to the present invention is to be immobilized, this may be carried out by immobilizing all antibodies in the sample or by use of a class-capture reagent which immobilizes isotype-specific antibodies.

In another preferred embodiment, the prognosis, diagnosis, methods or test kit in line with the inventive teachings contemplate the use of indirect immunofluorescence. The person skilled in the art is familiar with such techniques and the preparation of suitable samples, which are described in the state of the art (US4647543; Voigt, J., Krause, C., Rohwäder, E, Saschenbrecker, S., Hahn, M., Danckwardt, M., Feirer, C., Ens, K, Fechner, K, Barth, E, Martinetz, T., and Stöcker, W. (2012), Automated Indirect Immunofluorescence Evaluation of Antinuclear Autoantibodies on HEp-2 Cells," Clinical and Developmental Immunology, vol. 2012, doi:10.1155/2012/65105; Bonilla, E., Francis, L., Allam, F., et al., Immunofluorescence microscopy is superior to fluorescent beads for detection of antinuclear antibody reactivity in systemic lupus erythematosus patients, Clinical Immunology, vol. 124, no. 1, pp. 18-21, 2007). Suitable reagents, devices and software packages are commercially available, for example from EUROIMMUN, Lübeck, Germany.

The invention provides a pharmaceutical composition comprising the inventive polypeptide, which composition is preferably suitable for administration to a subject, preferably a mammalian subject, more preferably a human. Such a pharmaceutical composition may comprise a pharmaceutically acceptable carrier. The pharmaceutical composition may, for example, be administered orally, parenterally, by inhalation spray, topically, by eyedrops, rectally, nasally, buccally, vaginally or via an implanted reservoir, wherein the term "parentally", as used herein, comprises subcutaneous, intracutaneous, intravenous, intramuscular, intra-articular, intrasynovial, instrasternal, intrathecal, intralesional and intracranial injection or infusion techniques. The pharmaceutical composition may be provided in suitable dosage forms, for example capsules, tablets and aqueous suspensions and solutions, preferably in sterile form. It may be used in a method of treatment of a disease, which method comprises administering an effective amount of the inventive polypeptide to a subject. In a preferred embodiment, the invention provides a vaccine comprising the inventive polypeptide, optionally comprising an auxiliary agent such as an adjuvans or a buffer, and the use of the inventive polypeptide for the preparation of a vaccine.

Within the scope of the present invention, a medical or diagnostic device comprising, preferably coated with the inventive polypeptide is provided. Preferably, such a medical or diagnostic device comprises the inventive polypeptide in a form that allows contacting it with an aqueous solution, more preferably the liquid human sample, in a straightforward manner. In particular, the inventive polypeptide may be immobilized on the surface of a carrier such as an artificial carrier, preferably selected from the group comprising glass plates or slides, biochips, microtiter plates, beads, for example magnetic beads, apharesis devices, chromatography columns, membranes or the like. Exemplary medical devices include blots, preferably line, Western or dot blots, microtiter plates, glass slides for microscopy, beads and biochips. In addition to the inventive polypeptide, the medical or diagnostic device may comprise additional polypeptides, preferably in an enriched, isolated and/or recombinant form, for example positive or negative controls or one or more known other antigens binding to autoantibodies of diagnostic value, particularly those related to other diseases associated with one or more identical or similar symptoms.

In addition to the inventive polypeptide, such medical or diagnostic device may comprise means for detecting one or more further autoantibodies, preferably one or more antibodies binding specifically to one or more, preferably 2, 3, 4, 5, 6, 7, 8, 9, 10 or 11 antigens from the group comprising Mi-2beta (data base entry: Q14839), Ku (p70 data base entry: P12956 , p80 data base entry: P13010), PM-Scl 100 (Q01780), PM-Scl75 (Q06265), Jo-1 (P12081), SRP (P61011), PL-7 (P26639), PL-12 (P49588), EJ (P41250), OJ (Q6P0M4), Ro-52 (P19474). Such means is preferably an polypeptide from the group comprising Mi-2beta, Ku, PM-Scl 100, PM-Scl75, Jo-1, SRP, PL-7, PL-12, EJ, OJ, Ro-52 or a variant thereof, which variant preferably binds to the respective autoantibody to said antigen. Such means and antigens are commercially available, and their preparation is described in the state of the art. For example, the product "EUROLINE Myositis Antigens Profile 3 (IgG)" (Product number DL 1530-1601-3 G) comprises all these antigens, and the manual, which is incorporated by reference in its entirety, lists relevant literature.

The inventive teachings provide a kit, preferably for diagnosing a disease. Such a kit may comprise instructions detailing how to use the kit and a means for contacting the inventive polypeptide with a bodily fluid sample from a subject, for example a medical device according to the present invention. Furthermore, the kit may comprise a positive control, for example a batch of autoantibody or recombinant antibody known to bind to the inventive polypeptide and a negative control, for example a protein having no detectable affinity to the inventive polypeptide such as bovine serum albumin. Finally, such a kit may comprise a standard solution of the antibody or antigen for preparing a calibration curve.

In a preferred embodiment, the kit comprises a means for detecting an autoantibody, binding to the inventive polypeptide, preferably by detecting a complex comprising the inventive polypeptide and an antibody binding to the inventive polypeptide. Such means is preferably an agent that binds to said complex and modifies the complex or carries a label such that makes the complex detectable. For example, said means may be a labeled antibody binding to said polypeptide, at a binding site other than the binding site recognized by the primary antibody or to a constant region of the primary antibody. Alternatively, said means may be a secondary antibody binding to the constant region of the autoantibody, preferably a secondary antibody specific for mammalian IgG class of antibodies.

The inventive polypeptide comprising SEQ ID NO1 or a variant thereof may be produced or provided in the form of a cell comprising and/or expressing a nucleic acid encoding said polypeptide. In a preferred embodiment, the nucleic acid is a recombinant nucleic acid or part of a nucleic acid, and is, in a more preferred embodiment, part of a vector, in which it may be functionally linked with a promoter that allows for expression, preferably inducible overexpression of the nucleic acid. The person skilled in the art is familiar with a variety of suitable vectors, which are commercially available, for example from Novagen For example, a vector encoding for fusion constructs with a C-terminal GFP may be used. The cell may be a eukaryotic or prokaryotic cell, preferably of eukaryotic cell, such as a yeast cell, and is more preferably a mammalian, more preferably a human cell such as a HEK293 cell. Examples of a mammalian cell include a HEK293, CHO or COS-7 cell. The cell comprising the nucleic acid encoding for the inventive polypeptide may be a recombinant cell or an isolated cell, wherein the term "isolated" means that the cell is enriched such that, compared to the environment of the wild type of said cell, fewer cells of other differentiation or species or in fact no such other cells are present.

In a preferred embodiment, the nucleic acid or vector according to the present invention is codon optimized and/or is expressed in a cell with a codon preference altered such that the level of tRNAs is increased, which tRNAs recognize codons that are naturally rare in this cell. In a preferred embodiment, the term "codon optimized" means that the nucleic acid sequence from the original source, for example from a human, is subjected to targeted mutagenesis to remove codons that are rare in the cell in which the nucleic acid is to be expressed. SEQ ID NO6 encodes a codon-optimized nucleic acid according to the present invention. The optimization strategy also reduced of the GC-content especially in the 5' region of SEQ ID NO15. Suitable cells and nucleic acids are commercially available and described in the state of the art, for example in Burgess-Brown, N. A., Sharma, S., Sobott, F., Loenarz, C., Oppermann, U., and Gileadi, O. (2008): Codon optimization can improve expression of human genes in Escherichia coli: a multi-gene study, Protein Expr Purif., 29; 94-102.

The inventive teachings may not only be used for a diagnosis, but also for preventing or treating a disease, more specifically a method for preventing or treating a disease, comprising the steps a) reducing the concentration of autoantibodies binding to the inventive polypeptide in the subject's blood and/or b) administering one or more immunosuppressive pharmaceutical substances, preferably selected from the group comprising rituximab, prednisone, methylprednisolone, cyclophosphamide, mycophenolatemofetil, intravenous immunoglobulin, tacrolimus, cyclosporine, methotrexate, azathioprine and/or the pharmaceutical composition.

The present invention is further illustrated by the following figures and non-limiting sequences and examples from which further features, embodiments, aspects and advantages of the present invention may be taken.
**SEQ ID N01 (immunogenic wild type sequence of cN-1A):**
**SEQ ID NO2 (major epitope 1 of cN-1A):**
   PVWEEAKIFYDNLAPKKKPKSPK
**SEQ ID NO3 (major epitope 2 of cN-1A):**
   SERIVKAHGLDRFFEHEKAHENK
**SEQ ID NO4 (major epitope 3 of cN-1A):**
   AHVPYGVAQTPRRTAPAKQAPSA
**SEQ ID NO5 (immunogenic wild type sequence of cN-1A having a lower degree of enzymatic activity):**
**SEQ ID NO6 (codon-optimized nucleic acid encoding sequence of cN-1A):**
**SEQ ID NO7: pET24d-Mup44**
**SEQ ID NO8: pET24d-Mup44 [D211A]**
**SEQ ID NO9: pET24d-Mup44 (opt.)**
**SEQ ID NO10: pET24d-Mup44 (opt.) [D211A]**
**SEQ ID NO11: pTriEX-Mup44**
**SEQ ID NO12: pTriEX-Mup44 [D211A]**
**SEQ ID NO13: pTriEX-Mup44 (opt.):**
**SEQ ID NO14: pTriEX-Mup44 (opt.) [D211A]**
**SEQ ID NO15: 5' region of the nucleotide sequence encoding cN-1A**

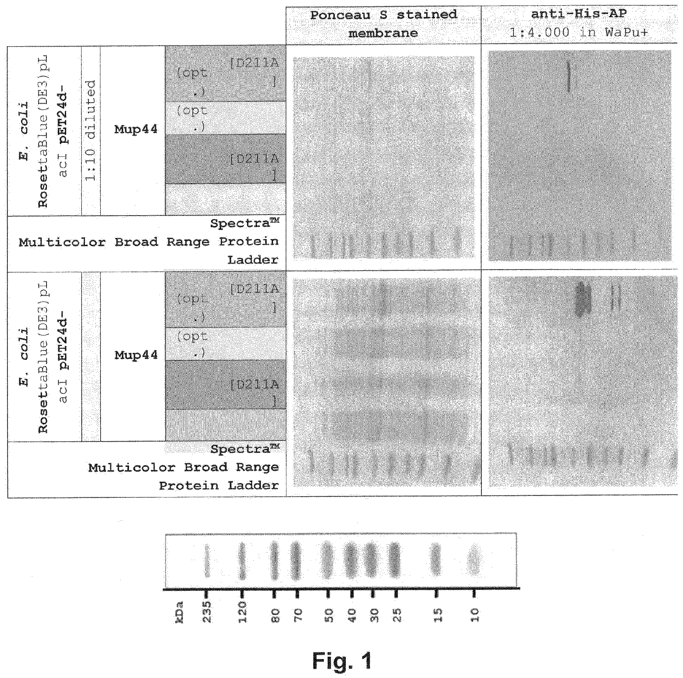

**Fig. 1** shows the results of Ponceau S staining and Western blot analysis of Mup44 expression in *E. coli* transfected with pET24d-Mup44, pET24d-Mup44 [D211A], pET24d-Mup44 (opt.) and pET24d-Mup44 (opt.) [D211A], respectively. The lysates are normalized according to cell culture density. In the upper part of the Westernblot 1:10 diluted samples were used.

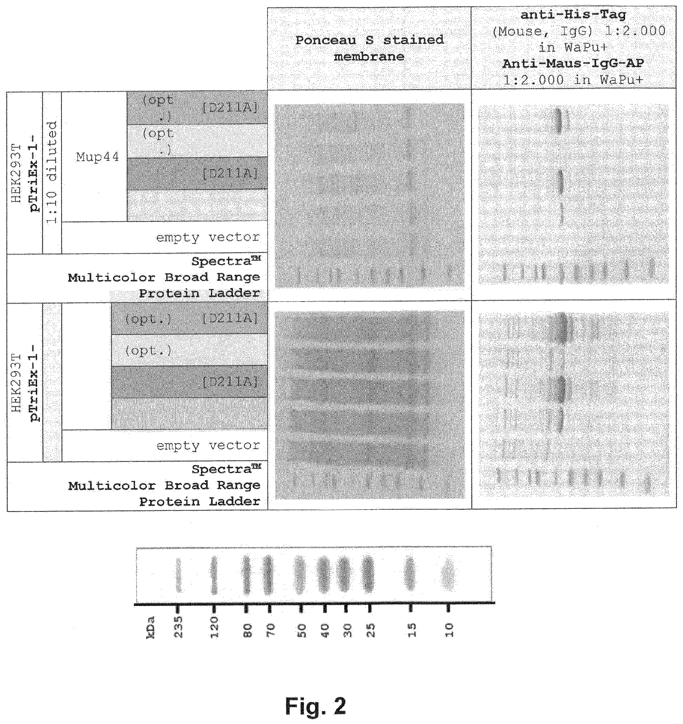

**Fig. 2** shows the results of Ponceau S staining and Western blot analysis of Mup44 expression in HEK293T cells transfected with pTriEx-Mup44, pTriEx-Mup44 [D211A], pTriEx-Mup44 (opt.) and pTriEx-Mup44 (opt.) [D211A], respectively. The cellular fraction normalized according to cell culture density were analyzed. In the upper part of the Westernblot 1:10 diluted samples were analyzed.

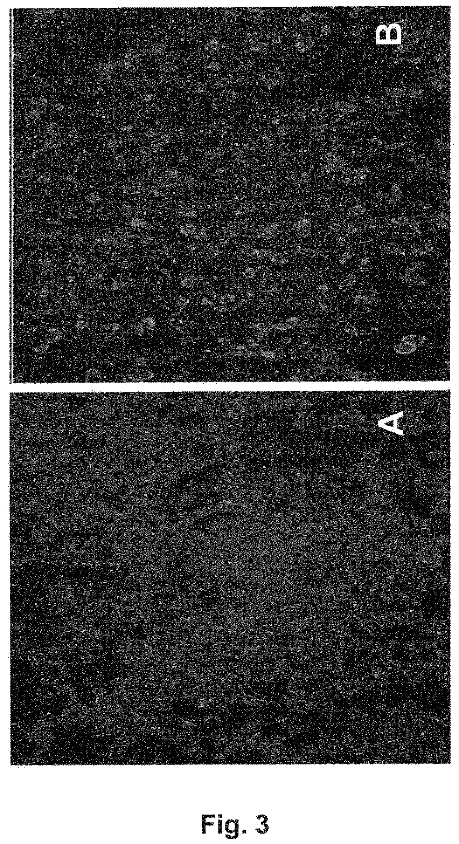

**Fig. 3** shows the results of immunofluorescence staining of recombinant Mup44 mutant expression in HEK293T cells transfected with pTriEx empty vector as a negative control (panel **A**) or pTriEx-Mup44 [D211A] (panel **B**).

### Examples:

### Example 1: Cloning

Mup44, Mup44 [D211A], Mup44 (opt.) and Mup44 (opt.) [D211A] were cloned into E. *coli* expression vector pET24d and mammalian expression vector pTriEx according to standard cloning protocols, respectively, yielding vectors pET24d-Mup44 (SEQ ID NO7), pET24d-Mup44 [D211A] (SEQ ID NO8), pET24d-Mup44 (opt.) (SEQ ID NO9) and pET24d-Mup44 (opt.) [D211A] (SEQ ID NO10), pTriEx-Mup44 (SEQ ID NO11), pTriEx-Mup44 [D211A] (SEQ ID NO12), pTriEx-Mup44 (opt.) (SEQ ID NO13) and pTriEx-Mup44 (opt.) [D211A] (SEQ ID NO14). Both vectors encode a C-terminal His-Tag fused to the protein of interest.

### Example 2: Analysis of expression efficiency of four Mup44 constructs in E. coli by Western blotting

*E. coli* RosettaBlue (DE3) pLacI was transformed with pET24d-Mup44, pET24d-Mup44 [D211A], pET24d-Mup44 (opt.) and pET24d-Mup44 (opt.) [D211A] and cells were selected using Kanamycin/Cam at a concentration of 50 and 34 µg/ml, respectively.

Transformed *E. coli* cells were used to induce the recombinant protein according to standard procedures. Briefly, cells were grown in LB-Medium supplemented with 50 µg/ml Kanamycin and 34 µg/ml Chloramphenicol. The optical density of the culture at 600nm was estimated and adjusted to 0.6 (d=1cm) in fresh LB medium Kanamycin/Chloramphenicol/IPTG (50µg/ml, 34 µg/ml, 2 mmol/l). This cultures were incubated for 3 hours at 37°C with shaking (100 rpm). At the end the optical density at 600nm was measured and OD normalized samples were spun down at 21000 rcf at room temperature for 5 minutes). *E. coli* cell pellets were resuspended in 50 µl 0.1 % SDS solution and 17 µl 4-fold NuPAGE-P.P. supplemented with50 mmol/l DTT was added to the cells. The samples were subsequently incubated at 70 °C for 10 minutes and further lysed by by ultrasonication. 15 µl and 1,5 µl of the lysate was loaded to an NuPAGE™ Novex™ 4-12% Bis-Tris Protein Gels, 1.0 mm, 17-well (Thermo Fisher, NP0329) gel. NuPAGE MES SDS Running Buffer was used for electrophoresis .

The gel was removed from the plastic cassette and the proteins were electroblotted onto Amersham-Protran 0,2 µm nitrocellulose membrane (GE Healthcare Life Sciences, 10600002) using a Hoefer TE22 transfer tank blotter using NuPAGE transfer buffer (Thermo Fisher, NP0006) according to the instruction of the manufacturer but without methanol.

Membrane bound proteins were reversible stained with 0.2% (w/v) Ponceau S solution in 7% (v/v) acetic acid to verify proper transfer. After documentation the Ponceau S stain was washed out using 50 mM Tris base solution and the membrane was protein saturated (blocked) by an 10 minute incubation in milk protein containing Wash buffer plus (EUROIMMUN).

The detection of His-Tag fusion proteins in bacterial lysate was done employing a 1:4000 dilution of the mouse Monoclonal Anti-polyHistidine-Alkaline Phosphatase antibody (Sigma-Aldrich, A5588) in Wash buffer plus (EUROIMMUN) followed by three extensive washing steps. Staining was performed by incubation with Alkaline phosphatase substrate solution (Nitroblue tetrazolium chloride/5-Bromo-4-chloro-3-indolylphosphate (NBT/BCIP), EUROIMMUN).

**Fig. 1** shows that *E. coli* transformed with pET24d-Mup44 (opt.) [D211A] expressed Mup44, whereas there was hardly any expression of other constructs.

### Example 3: Analysis of expression efficiency of four Mup44 constructs in HEK293T by Western blotting

HEK293T cells were transfected with pTriEx-Mup44, pTriEx-Mup44 [D211A], pTriEx-Mup44 (opt.) and pTriEx-Mup44 (opt.) [D211A], respectively.

HEK293T cells transfected with the pTriEx constructs in 2cm² wells were resuspended in the corresponding 0.5 ml D-MEM-10% FBS medium and spun down in at 21000 rcf at room temperature for 5 minutes. Cell pellets (Z) were resuspended in 50 µl 0.1% SDS solution and 17 µl 4-fold NuPAGE-P.P. supplemented with 50 mM DTT was added to the lysate. The cells were subsequently incubated at 70 °C for 10 minutes and further lysed by ultrasonification. 15 µl and 1,5 µl of each cell lysate was used for His-Tag westenblot essentially as decribed in Example 2 with to following modification.

For the detection of His-Tag fusion proteins in HEK293 lysates the membrane was incubated with anti-His-Tag mAb (Merck, 70796) 0.1 µg/ml in Wash buffer plus for 30 minutes followed by three extensive washing steps and a further 30 minutes incubation with anti-Maus IgG-Alkaline phosphatase (Dako, D0486) 1:2000 in wash buffer plus (EUROIMMUN).

**Fig. 2** shows that HEK293T cells transfected with pTriEx-Mup44 (opt.) [D211A] expressed Mup44, whereas there is hardly any expression of other constructs.

### Example 4: Analysis of expression of four Mup44 constructs in HEK293T cells by immunofluorescence staining

HEK293T cells were transfected with pTriEx empty vector and pTriEx-Mup44[D211A], respectively, fixed with acetone and subsequently stained with 1 µg/ml anti-His-Tag mAb (Merck, 70796) in IFT sample buffer (EUROIMMUN) and a fluorescein-conjugated monoclonal sheep anti-mouse IgG antibody (Sigma, F6257-2ML, 1:200 diluted in IFT sample buffer, EUROIMMUN) (**Fig. 3**). Empty pTriEx vector was used as a negative control.

**Fig. 3** shows that Mup44[D211A] could be detected in samples using pTriEx-Mup44[D211A] transfected HEK293T cells by immunofluorescence staining.

## Claims

1. A polypeptide comprising a stretch of at least 150 successive amino acids of cN-1A according to SEQ ID NO1 or a variant thereof, which polypeptide has the ability to bind specifically to an autoantibody to SEQ ID NO1 from an inclusion body myositis (IBM) patient, wherein the polypeptide or the variant comprises a mutation at Aspartate 211 and the Aspartate 211 is replaced by an amino acid residue other than glutamate, wherein the variant is at least 80% identical to the sequence according to SEQ ID NO1.

2. The polypeptide according to claim 1, wherein said polypeptide comprises at least one sequence from the group comprising SEQ ID NO2, SEQ ID NO3 and SEQ ID NO4 or a variant thereof reactive to autoantibodies to SEQ ID NO1.

3. The polypeptide according to any of claims 1 to 2, wherein the polypeptide
(a) is a recombinant polypeptide; and/ or
(b) is optimized in terms of codon usage; and/or
(c) is immobilized.

4. A nucleic acid encoding the polypeptide according to any of claims 1 to 3 or a vector comprising said nucleic acid or a cell comprising said nucleic acid or said vector.

5. The cell according to claim 4, wherein the cell is a Gram negative bacterial cell or a mammalian cell.

6. A method comprising the step a) cultivating the cell according to any of claims 4 to 5 under conditions compatible with the expression of the polypeptide according to any of claims 1 to 3 and producing the polypeptide according to any of claims 1 to 3.

7. The method according to claim 6, further comprising step b) purifying the polypeptide according to any of claims 1 to 3.

8. The method according to any of claims 6 to 7, further comprising step c) immobilizing the polypeptide according to any of claims 1 to 3.

9. A diagnostically or therapeutically useful carrier comprising the polypeptide according to any of claims 1 to 3 or the cell according to any of claims 4 to 5, which carrier is preferably selected from the group comprising a glass plate or slide, biochip, microtiter plate, bead, preferably magnetic bead, apharesis device, chromatography column, membrane and blot, preferably line blot, Western blot or dot blot.

10. A kit comprising the polypeptide according to any of claims 1 to 3, the cell according to any of claims 4 or 5 or the carrier according to claim 9.

11. A use of the polypeptide according to any of claims 1 to 3, the cell according to any of claims 4 to 5 or the carrier according claim 9 for diagnosing a disease in vitro, wherein the disease is an autoimmune disease **characterized by** the presence of autoantibodies to SEQ ID N01.

12. A polypeptide according to any of claims 1 to 3, a cell according to any of claims 4 to 5 or a carrier according claim 9 for use in an in vivo method of diagnosis, wherein the disease is inclusion body myositis (IBM).

13. A pharmaceutical composition comprising the polypeptide according to any of claims 1 to 3, the cell according to any of claims 4 to 5 and preferably a pharmaceutically acceptable carrier.

## Patentansprüche

1. Ein Polypeptid, das einen Abschnitt von mindestens 150 aufeinanderfolgenden Aminosäuren von cN-1A gemäß SEQ ID NO1 oder eine Variante davon umfasst, wobei das Polypeptid die Fähigkeit hat, spezifisch an einen gegen SEQ ID NO1 gerichteten Autoantikörper von einem Patienten mit Einschlusskörpermyositis (IBM) zu binden, wobei das Polypeptid oder die Variante davon eine Mutation an Aspartat 211 umfasst und wobei das Aspartat 211 durch einen anderen Aminosäurerest als Glutamat ersetzt ist, und wobei die Variante mindestens 80% identisch zu der Sequenz gemäß SEQ ID NO1 ist.

2. Das Polypeptid nach Anspruch 1, wobei dieses Polypeptid mindestens eine Sequenz aus der Gruppe umfassend SEQ ID NO2, SEQ ID NO3 und SEQ ID NO4 oder eine Variante davon, die mit einem Autoantikörper gegen SEQ ID NO1 reagiert, umfasst.

3. Das Polypeptid nach einem der Ansprüche 1 bis 2, wobei das Polypeptid
(a) ein rekombinantes Polypeptid ist; und/oder
(b) in Bezug auf Codonverwendung optimiert ist; und/oder
(c) immobilisiert ist.

4. Eine Nukleinsäure, die das Polypeptid nach einem der Ansprüche 1 bis 3 kodiert, oder ein Vektor, der diese Nukleinsäure umfasst, oder eine Zelle, die diese Nukleinsäure oder diesen Vektor umfasst.

5. Die Zelle nach Anspruch 4, wobei die Zelle eine gramnegative Bakterienzelle oder eine Säugetierzelle ist.

6. Ein Verfahren umfassend den Schritt a) Kultivieren der Zelle nach einem der Ansprüche 4 bis 5 unter Bedingungen, die mit der Expression des Polypeptids nach einem der Ansprüche 1 bis 3 und der Herstellung des Polypeptids nach einem der Ansprüche 1 bis 3 vereinbar sind.

7. Das Verfahren nach Anspruch 6, ferner umfassend Schritt b) Reinigen des Polypeptids nach einem der Ansprüche 1 bis 3.

8. Das Verfahren nach einem der Ansprüche 6 bis 7, ferner umfassend Schritt c) Immobilisieren des Polypeptids nach einem der Ansprüche 1 bis 3.

9. Ein diagnostisch oder therapeutisch verwendbarer Träger, der das Polypeptid nach einem der Ansprüche 1 bis 3 oder die Zelle nach einem der Ansprüche 4 bis 5 umfasst, wobei der Träger bevorzugt aus der Gruppe ausgewählt ist, die eine Glasplatte oder einen Objektträger, einen Biochip, eine Mikrotiterplatte, einen Bead, bevorzugt einen magnetischen Bead, eine Apharese-Vorrichtung, eine Chromatographie-Säule, eine Membran und einen Blot, vorzugsweise einen Linienblot, Western Blot oder Dot Blot, umfasst.

10. Ein Kit, das das Polypeptid nach einem der Ansprüche 1 bis 3, die Zelle nach einem der Ansprüche 4 oder 5 oder den Träger nach Anspruch 9 umfasst.

11. Eine Verwendung des Polypeptids nach einem der Ansprüche 1 bis 3, der Zelle nach einem der Ansprüche 4 bis 5 oder des Trägers nach Anspruch 9 für die in vitro-Diagnose einer Krankheit, wobei die Krankheit eine Autoimmunerkrankung ist, die durch das Vorhandensein von Autoantikörpern gegen SEQ ID NO1 gekennzeichnet ist.

12. Ein Polypeptid nach einem der Ansprüche 1 bis 3, eine Zelle nach einem der Ansprüche 4 bis 5 oder ein Träger nach Anspruch 9 zur Verwendung in einem in vivo-Diagnoseverfahren, wobei die Krankheit Einschlusskörpermyositis (IBM) ist.

13. Eine pharmazeutische Zusammensetzung, die das Polypeptid nach einem der Ansprüche 1 bis 3, die Zelle nach einem der Ansprüche 4 bis 5 und bevorzugt einen pharmazeutisch verträglichen Träger umfasst.

## Revendications

1. Polypeptide comprenant un tronçon d'au moins 150 acides aminés successifs de cN-1A selon SEQ ID n° 1 ou un variant de celui-ci, lequel polypeptide est apte à se fixer spécifiquement à un auto-anticorps anti-SEQ ID n° 1 issu d'un patient souffrant d'une myosite à corps d'inclusion (IBM), où le polypeptide ou le variant comprend une mutation au niveau de l'aspartate 211 et l'aspartate 211 est remplacé par un résidu d'acide aminé autre que le glutamate, où le variant possède une identité d'au moins 80% par rapport à la séquence selon SEQ ID n° 1.

2. Polypeptide selon la revendication 1, où ledit polypeptide comprend au moins une séquence choisie dans le groupe constitué par SEQ ID n° 2, SEQ ID n° 3 et SEQ ID n° 4, ou un variant de celle-ci qui est réactive vis-à-vis des auto-anticorps anti-SEQ ID n° 1.

3. Polypeptide selon l'une quelconque des revendications 1 à 2, où le polypeptide
(a) est un polypeptide recombiné ; et/ou
(b) est optimisé en termes d'usage de codon ; et/ou
(c) est immobilisé.

4. Acide nucléique codant pour le polypeptide selon l'une quelconque des revendications 1 à 3, ou un vecteur comprenant ledit acide nucléique ou une cellule comprenant ledit acide nucléique ou ledit vecteur.

5. Cellule selon la revendication 4, où la cellule est une cellule bactérienne à Gram négatif ou une cellule mammalienne.

6. Méthode comprenant l'étape a) de culture de la cellule selon l'une quelconque des revendications 4 à 5 dans des conditions compatibles avec l'expression du polypeptide selon l'une quelconque des revendications 1 à 3 et la production du polypeptide selon l'une quelconque des revendications 1 à 3.

7. Méthode selon la revendication 6, comprenant en outre l'étape b) de purification du polypeptide selon l'une quelconque des revendications 1 à 3.

8. Méthode selon l'une quelconque des revendications 6 à 7, comprenant en outre l'étape c) d'immobilisation du polypeptide selon l'une quelconque des revendications 1 à 3.

9. Support utile sur le plan du diagnostic ou de la thérapie, comprenant le polypeptide selon l'une quelconque des revendications 1 à 3 ou la cellule selon l'une quelconque des revendications 4 à 5, lequel support est choisi de préférence dans le groupe constitué par une plaque ou une lamelle de verre, une puce biologique, une plaque de microtitration, une bille, de préférence une bille magnétique, un dispositif d'aphérèse, une colonne de chromatographie, une membrane et un buvardage, de préférence un immunodosage en ligne, un buvardage de Western ou un transfert en point.

10. Kit comprenant le polypeptide selon l'une quelconque des revendications 1 à 3, la cellule selon l'une quelconque des revendications 4 ou 5 ou le support selon la revendication 9.

11. Utilisation du polypeptide selon l'une quelconque des revendications 1 à 3, de la cellule selon l'une quelconque des revendications 4 ou 5 ou du support selon la revendication 9, pour le diagnostic d'une maladie *in vitro,* où la maladie est une maladie auto-immune **caractérisée par** la présence d'auto-anticorps anti-SEQ ID n° 1.

12. Polypeptide selon l'une quelconque des revendications 1 à 3, une cellule selon l'une quelconque des revendications 4 ou 5 ou un support selon la revendication 9, pour une utilisation dans une méthode de diagnostic *in vivo,* où la maladie est la myosite à corps d'inclusion (IBM).

13. Composition pharmaceutique comprenant le polypeptide selon l'une quelconque des revendications 1 à 3, la cellule selon l'une quelconque des revendications 4 ou 5, et de préférence, un véhicule pharmaceutiquement acceptable.
